# EUROPEAN PATENT APPLICATION

(11) **EP 2 495 318 A1**
(43) Date of publication of application: **05.09.2012**
(21) Application number: 10826885.5
(22) Date of filing: 29.10.2010
(51) Int. Cl.: C12N 15/00, C12N 15/09, C12Q 1/68

(54) **PROBE FOR DETECTING MPL GENE POLYMORPHISM AND USE OF THE SAME**

(30) Priority: 30.10.2009 JP 2009251429
(71) Applicant: Arkray, Inc., Minami-ku Kyoto-shi Kyoto 601-8045 (JP)
(72) Inventor: HIRAI Mitsuharu, Kyoto 602-0008 (JP); KOMORI Mariko, Kyoto 602-0008 (JP)
(74) Representative: Jones, Elizabeth Louise
(86) International application number: PCT/JP2010/069379
(87) International publication number: WO 2011/052755

(57) **Abstract**

The present invention provides a probe that can identify a polymorphism in the MPL gene easily and with high reliability and use of the probe. The probe used for detecting a polymorphism in the MPL gene is a probe containing any one of oligonucleotides (P1), (P1'), (P2), and (P2'), wherein:
(P1) is a 9- to 50-mer oligonucleotide composed of a base sequence including the 11535th to 11543rd bases in SEQ ID NO: 1 and having the 11543rd base in its 3' end region;
(P1') is an oligonucleotide composed of a base sequence complementary to the oligonucleotide (P1);
(P2) is a 10- to 50-mer oligonucleotide composed of a base sequence including the 11535th to 11544th bases in SEQ ID NO: 1 and having the 11544th base in its 3' end region; and
(P2') is an oligonucleotide composed of a base sequence complementary to the oligonucleotide (P2).

### [Sequence Listing]

SEQUENCE LISTING

<110>  Arkray, Inc.

<120>  Probe for detecting of polymorphism of MPL gene and use thereof

<130>  TF10046-00WO

<150>  JP 2009-251429
<151>  2009-10-30

<160>  13

<170>  PatentIn version 3.1

<210>  1
<211>  16661
<212>  DNA
<213>  Homo sapiens

<400>  1
cctgaaggga ggatgggcta aggcaggcac acagtggcgg agaagatgcc ctcctgggcc      60
ctcttcatgg tcacctcctg cctcctcctg gcccctcaaa acctggccca agtcagcagc     120
caaggtgagg tgcacagagg gtggagatca cctatgccca ggaagaggga gccctgggag     180
gtgatgcagg gccccgggag gggaggtaga gtaagaggct ctcctgctgg tcccctcccc     240
ttccacataa acatgcctgg gaggacccag ggccaactca ccagctgttc cttagatgtc     300
tccttgctgg catcagactc agagcccctg aagtgtttct cccgaacatt tgaggacctc     360
acttgcttct gggatgagga agaggcagcg cccagtggga cataccagct gctgtatgcc     420
tacccgcggt aggtgctgga ctgtgcccca ctccccatgt atctgtccct gcacttagct     480
gagtcccact ccagcagctt tcctgcctgt ccgaggacca ctctgaatac aagccctaag     540
tacctacttt ttgaacagat gtgctttgga tgtatgtggg ccccagctcc agccctacat     600
aacccctaat cccacctatc ccaggcagtg agaagaaaaa tggcagtact agagacagaa     660
gttgggcatg ggcccaggtc tgggtcctca gggctccgca tggtggctgt gtaggaggga     720
cctcttctat gccaacaggg agaagccccg tgcttgcccc ctgagttccc agagcatgcc     780
ccactttgga acccgatacg tgtgccagtt tccagaccag gaggaagtgc gtctcttctt     840
tccgctgcac ctctgggtga agaatgtgtt cctaaaccag actcggactc agcgagtcct     900
ctttgtggac agtgtaggta agagccatcc tcctgtcacc ctgccccctc cacttgctgc     960
ccccagtcca gctcccggaa tcagacttgc ctgtgccctt ccagctcagc acggataccc    1020
ctattaccag accccctgag gcaccccaag actcccttgt cattcctccc agccttggca    1080
tctagagcta gaaatgccca acagatcatt cacaccctgt tcctcccatc gtcaacaaat    1140
catttattca tccattcaag agttacagaa tacctactgt gtgccagaca ctgggctagg    1200
tgatggggat atggagagaa aaagggatcc ctgcttacaa gaagcactga gtctagcaag    1260
ccatcatact gcaaagaagt cactgttctc atgagagaag aacagaatca ggagcctccc    1320
tagcctgccc tccaggagaa tgacagccta caatttttga atccagaagc tgccccaatt    1380
cagcccccag cacccctctc tgcagtccag aggctgagcc atagactgtg gtactcagag    1440
ttctgatgtg ccctgtcttg ccctcaggcc tgccggctcc ccccagtatc atcaaggcca    1500
tgggtgggag ccagccaggg gaacttcaga tcagctggga ggagccagct ccagaaatca    1560
gtgatttcct gaggtacgaa ctccgctatg gccccagaga tcccaagaac tccactggtc    1620
ccacggtcat acagctgatt gccacagaaa cctgctgccc tgctctgcag aggcctcact    1680
cagcctctgc tctggaccag tctccatgtg ctcagcccac aatgccctgg caagatggac    1740
caaagcagac ctccccaagt agagaagtat gctgaccttc ttctgcccca cctcttatct    1800
cctaccttca atcttgcccc aggaaaggac agaccatact ttggggattc cagacctagg    1860
ttcgtccttg gagagttagt ataggctcag atatgagcca cgcctactta ggggcttcct    1920
actttgggtc attcccactg acaaaagcaa aggctttcag gcctccaaat taatggagat    1980
ttcgcaacaa aaccctgaac accacctgaa acccaccaac ttagcccctg gtctgtgtgc    2040
atatctatcc agcaaggagc tgagctcctc tccacagaga tgctgtgcaa atataagggt    2100
tggaggctct ctcagctgac aggcagacct agattgtgaa gctgggattt tcctcccaag    2160
gcttcagctc tgacagcaga gggtggaagc tgcctcatct caggactcca gcctggcaac    2220
tcctactggc tgcagctgcg cagcgaacct gatgggatct ccctcggtgg ctcctgggga    2280
tcctggtccc tccctgtgac tgtggacctg cctggagatg caggtgagtc aacaaaggaa    2340
tagggagatg gggaggagat aaaagaatat ctctagggaa gcctgggcta gatctgaagc    2400
tctgggaacc atggtccttc ctgatgatct cgaacttgcc actggacagg aactatgttc    2460
agggaaagaa gagagaatag gagtcaatgt tctaagttat atgtgtagaa attatctgaa    2520
atctgaacac cctatacagt aggggcacac gggccctgat gggacttact tctttgactt    2580
tagtggcact tggactgcaa tgctttacct tggacctgaa gaatgttacc tgtcaatggc    2640

agcaacagga ccatgctagc tcccaaggct tcttctacca cagcagggca cggtgctgcc 2700
ccagagacag gtgagagctg aactgctgat tgaggttggt gtcatgggag tgagccacaa 2760
tcttgcagaa aaaaagaaga gagtgttctt ggtcctcttc actctccttc ctttgtctcc 2820
aaaccataca gctttcaatg ctctcttatc tattctgtca tcctccaatc gatattttat 2880
cttctcaacc cctctctgtt cccattggga atgctttggt ttagtttagc cttaatcatg 2940
tcactgggac cattgcaaca tcctacagtc taattcacct ccaatgtatt ccccatgctg 3000
ctaacagagt gatctttgtt aagctcaaac tgtgcatgac ctttgtaaag ctctaacttt 3060
gcatagtctc cccttactca aacatgtact gcaatttctc attatcaaag ccataaaaaa 3120
tttagcctgg ccttcaaaga cttccaccaa gcacctcatt gattcttcat cctctccata 3180
tctaaatcta taaccaagtt gtattgattt tacctattaa ctatctctag aatctgccca 3240
cttgtctatc actgccatta tcctactaca acccatcatt aattctctct tgggctactg 3300
aaaagacata gtaactgatc accctgagtc ttccaagtaa ttcttcacat aggtgctgaa 3360
gggatctttt cattttgttt tgttttgaga cagtctggct ctgttgccca ggctggaatg 3420
caatagtgca atcttggctc actgcaacct ccacctcccg ggatcaaggg attctcctgc 3480
ctcagcctcc caaggagctg ggattacagg catgtgccac cataccccat taattatttt 3540
tgtatttagt agagaggggg cttcaccatg ttggtcaggc tggtctcgaa ctcctgacct 3600
caggtgatcc acctgccttg gcctcccaaa gtgctgggat tacaggtgtg agccaccgca 3660
cctagcctga agggaccttt taaaagtgca aaaatgaatg taacactccc tgcttaaaaa 3720
tcttcaatag cttcccacta gtcttaggat aaagaaaaaa acctctacca tggacttgaa 3780
aagttctgag actcagcccc tacgtacttc ttcagcttca tcttgtgcta tgctgccctt 3840
tgatccccat gctccagcac agggcttctg tggtcccctc atatctggca tattcccttc 3900
tgcttctagg tctttacact gctgtttacc cagccctact gcctttactt aacttctact 3960
tatcctccaa atctcaactc aagcgtcact ttcttaggga ccctcccctg gccttcttta 4020
ttaagtccaa tagcactaat atacattctt gttgtaccat gtaccgcttc ttcttcttct 4080
tttttttttt cttttttttt tctttttgag acagagtctc actctgtcgc ccaggctgga 4140
gggcagtggt gccatctcgg ctcaccgcaa gccccgcctc ccgggttcat gccattctcc 4200
tgcctcagcc tcccaagtag ctgggactac aggtgcccgc caccacgcct ggctaatttt 4260
ttgtattttt agtagagatg gggtttcact gtaagtgtta gccaggatag tctcgatctg 4320
acctcgtgat ccacccacct cggcctccca aagtgccggg attacaggcg tgagccacca 4380
tgcctggccg tacctctcct tcttaagtac ttatcatagt catactttca tatttatagt 4440
ttatatgatt atttggttga taataaaaat ctggccaggc ccagtggctc acacctgtaa 4500
tcccagcact ttgggagact aaggtgagaa gatcacttga ggccaggagt ttgagaccag 4560
cctagacaac atagtgagac ttcgtctcta caaaatttct ttaattagcc aggcatggtg 4620
gcacctgtat tcctagctac ttgggaggct aaggtgggag gatggcatga gcctaggagt 4680
ttgaggttac agtgagctat gattgcacca ctgcactcca gcctgggcaa cagatcaaga 4740
ctttgtctca aaaaaaacaa atctctcaga actccagttg agaaaggctg atcttgacca 4800
catatgatcc atgagggcaa gatcaatggg tttttattca ccaatatatt ctcacctacc 4860
aatagtaggt gcctggtaaa aaattgttga acactattaa ctatttaaat gtattcttcc 4920
agtttttttc tatgtattta catacatctc tatacaacat ttaaatttta ttatttttga 4980
gatagggtct tgctctgttg tccaggctgg agtgcagtgg catgattatg gctcactgca 5040
gcctcgacct cccagactca aacgatcctc ccacctgagc ctcctgatta gctaggacta 5100
caggcatttt ctttctttct ttctttcttt cttttttttt ttttttaagt agagacaagg 5160
tctccctatg ttgcccagac tggtctcaaa ctcctgagct caagcaatcc tcccatctca 5220
gcccctctac cacgcccagc ctaacatgta atattttttt tttttttgag atggagtctt 5280
gctctgttgc ccaggctgga gtgcagtggt gtgatctcag ctcactgcaa cctccgcctc 5340
cagggttcaa tcgattctcc tgcctcagcc ccccgagtag ctgggattat agatgtgcga 5400
caccatgccc agctaatttt ttgtattttt agtagagacg aggtttcatc atgttagcca 5460
ggctggtctc gaactcttga cctcaagtga tccacccacc tcagcctccc aaagtgctgg 5520
gattacaggt atgagccacc acacccagcc taatatttaa tttttaaata taaattggat 5580
tacattacat atatggtttg ttgactcacc tttttgctta aaaacatgtc tcagagatca 5640
ttacaaaaag atgtaactca ttctttttaa taactgcata catatccata ggattgagat 5700
gcctgagtgg actaaagggt agcaaaactg aagtcatcat ttcctctatc aaaacttgct 5760
tttcctgctg ggcacagtgg ctcacacctg taatcccagc actttgggag gccgaggtgg 5820
gtggatcacc tgaggtcagc agttggaaac cagcctggcc aacatggtga aacctcatca 5880
ttactaaaaa tacaaaaatt agtcgggtgt ggtggcacgc gcctgtagtc ccagcttctt 5940
gggaggctga ggcaggagaa atgcttgaac ccgggaagcg gaggttgcag tgagctgaaa 6000
tcgtgcccct gcactccagc ctgggcaaca gagagaagac tctgtctcaa aaaaaaagaa 6060
aaaaaaaact tgctttttct ttattgtctc cgatcctacc atgaattcag ttttcctgga 6120
ctgaaaaata gaaatcatcc tggacttctc ccattccaat accctccagg cctaaccaga 6180
aatagtcttg tcatttttac cttttcaata tctcttcaat catctctttc cctgtctctc 6240
actgctgcta ccctattttc acatcctcat cactttgcac atgaaaaact acaagaatgt 6300
cataatggat ttctctgctt ctagtcggcc ctcacaaatc ctttctacag actgtagtta 6360
gaatgatctt cctaaagtac aaaggtagct atggcacttc cctgcttaaa atccttaaat 6420
gtctacataa ctaccacaag gttagtagta attcaagttc aagctcccca gaaaggccat 6480
aaggccatcc atgacatggt ctgcagtctc acatttacca cctaccacca ggccctttat 6540

gcgaaaacca tctgcaccac ttagaatttc ccccaaagca ccacactctc ttatctatgc 6600
acaaccatgt gcctgggatg cctgtttgtc ccttttgcct atcctatttt tagttcaaaa 6660
ctcagtctaa acatcgccgt atccaggaag cattccctga ccctcccagt ctgggttgga 6720
tgctcccctt tgatgctccc aagccagtct atgcacatca cagaaaaata tcatgtttgt 6780
ttttctgtct ttcccactga aattagagct ctttgaacct tattcctcta tgcactcata 6840
gggcctggca cagtaccaat tggcacatag tatcaattat ttgttgatta aatgggtaaa 6900
catacccatt ttagtgacgg tgagccactt aggggttata atcaagaggg agacatgatt 6960
caattgttca ataaagaatt attgagagtg actttttgca aggtactggg gataaatagt 7020
gcaacaaata aaatggaaaa ggggccaggc tcggtggctc acacctgtaa tcccagcact 7080
ttgggaggcc aaggtgggca gatcacttga ggtcaggagt tcgagaccag cctggccaac 7140
atggcgaaac gccatctcta ctaaaaatac agaaattagt caggtgtggt ggtgggtgcc 7200
tgtaatccca gctactcagg aggctgaggc aggagaatcg cttggaccca agaggcagag 7260
gctgcagtga gctgagatcg taccattgca ctccagcctg ggcaacaaag caagactgtc 7320
tcaaaacatt tttaaaaata aataaagtgg aaagggttcc agcattgttg gggcttacat 7380
tagtagaggg gaggggaaaa ggagggggag aaaagtaagt aaagaaataa aggaacaaag 7440
ttataataga gactaaaggg gggcgtgtgg tgggtggggg gtgggtgttg ctaatttaga 7500
ctgagtggcc aggaaaagcc tcaccaggga ggtgacagat aagccgagat ctaaatggca 7560
agaaggaatg agtcacatga agacctacag tcagagcaat ccagggcaaa ggcaaagtgc 7620
aaagatagca catttggcat atctatggca cagaaagaag gcctgtgcgg atgaagggtg 7680
ataaactggc atgagaatca taagagatga gaatggcaag gcaaggagaa accaaatcac 7740
agagtcctgt aggcctggta aggggtttgg gttttattcc aagtgggatg aagagccact 7800
gaaggatttt aagcagtaaa caatgacaat ttgacttaca tttttaaaag atatgtttct 7860
ctgtgaataa ataaataaga gtagaagcag aatcaccagt cagaaatttg ccttaatcca 7920
agcaaggaat gatagtggct tcatctaaca tagcagtggt aaagatcaaa atcaatagtt 7980
tgtgacatta tattctggac attatcacat caacattttt ggaaacaaat tctggtagct 8040
gtgtggagaa caacttgaag gtagtggcaa aaacagaagc agaaaagcca gttaggagac 8100
tattgtcata atctaatagc aaaataacac ataggggaga gtaggaggaa tgacgtatga 8160
gaacacgtta aaaatagagg atatttttct ccagaaagat gagggttctt taatctttga 8220
aacaatcctg tgaggttggt atcattaacc cattttacaa atagaaggaa actggagcag 8280
agataaacca agtaatttgc ccaaagttac acaaaggtaa gccaagacct caactcttgg 8340
tgaaaaccca tgctgttttc acatatcatg ccaggtcttt cctcaaaggg aagaggccag 8400
acaacaaagc tgacttcaga aaacatcatg gcaaaatagc aaaaacaaac gaacaaatga 8460
acaaaggaaa aaacaaaaag aaaacatcat ggcataaaga cagagggaaa gctctggagg 8520
cccctggaag gaaggagtta aatgataatc cctgcaggcc atcgttcttg taggatggga 8580
agccttggga ttagtctctg gggcaggcct gattcaatga ctctgtgggg ctgggtctta 8640
ggtaccccat ctgggagaac tgcgaagagg aagagaaaac aaatccagga ctacagaccc 8700
cacagttctc tcgctgccac ttcaagtcac gaaatgacag cattattcac atccttgtgg 8760
aggtgaccac agccccgggt actgttcaca gctacctggg ctcccctttc tggatccacc 8820
aggctggtaa gaactttctt cctcattctt cccacatagt tcccaccccc actgaatctg 8880
accctgtgcc caggatcccc aactctgacc cttctgaccg atggctctgg tggcacaatg 8940
ccttgtgcac agaaggactt aagctgctcc ctgctgacat ccctgtagtg cgcctcccca 9000
ccccaaactt gcactggagg gagatctcca gtgggcatct ggaattggag tggcagcacc 9060
catcgtcctg ggcagcccaa gagacctgtt atcaactccg atacacagga gaaggccatc 9120
aggactggaa ggtatggtca agcaacaaat gcccacagac ctcactacgc aggggatccc 9180
tggggttggc catgcctgtt agcaggagtg aaagtgtcta tgtatccagt ctctgctagt 9240
atatctatgt ttatcagatt caactggtat cttagtctct cttgggcatc tgatatttct 9300
ggctactcct acttaacatt tctcttccct ttgtctttgg gatatcagtc ttccccgatt 9360
ttcctcctac ctctctgact gaactttctc actctcctct cctggctcct cctttccagc 9420
cttccacaaa atgctgtgct tcctagagtc tcatcctagg ctcttttctc ctctgataaa 9480
ataaatatgt tctccccagg tgagtgcttt aactccatgg tttcaatgtc catacattaa 9540
caatcgcaat tttaccattc taactcccat atggaaagga atttacaaac cacaggcaaa 9600
ttaccaaaat gtttgaagtc tgtttcctca ttggtgaaat gaggatgaag ctatctacct 9660
tatggagtta tagtaaagat taaatactat atctaaatac ctagcatact gcctggcata 9720
aggaaggtgc tcaatataca ttacttaaat attagtttgt ctacttttat tctcatttgc 9780
cacacatata tacaactatg tacaacatct tatggttcta ccaaaacttc ctccccctgg 9840
acatttcaga ctcagtctat ccagaatgga actcctcttc ctcctgaagc tttacttttt 9900
tattgtgata aaacttacat acaacaaagt gtacaaattt tacatatata gtctgtactt 9960
gtgtatacat ccacataacc ataaccagga tcaagatata aattatttcc agcacctcag 10020
aagctcccaa tgctccgaca catcccccgc caccaaaggt aatcaccatc ctgacattac 10080
cattgattca tttggactgt tgttgaactc tataaaatgg aaccctaaag tatatcttcc 10140
tcaaacttac ttctcttctg tgtcctgtct ctgacaatgg caccgctact tacccagtac 10200
ccaagcctag aagcaccctt gactcttctt gctcttccct tatcccttcc ccttactcat 10260
caactaagtt gatggcaata acgtactaat agtcctcaaa ttcctaccag gattgcctta 10320
gactcatctg tgaccatcca gccgtgtcat ccatgctttt agcacactaa actactctgt 10380
ggcacaaaac caagcattaa atgcttctct ggacttccgt ggtgccttgc actaattaat 10440

tgttgtgatt atttacctgt ttggatgtct ttctcattag atatgtaaga gacggtgttt 10500
tattgctttc tccccagtgc ctaacccaat gtttggagta tcacacagtt cataaatgtt 10560
tgctgccttc ctgcttgtac acatgatgct tgtaccccag cgcctaacgc aaatctggca 10620
tcctctgcag catgagtatt atttgtggca tggtctgtgt ggctctgaat atatctgttt 10680
ctgggggtgt ccatcgcccg acctggagtt gtgagaacac ccggtagggt gtgcgtgtac 10740
cgggatccct gccgagggtg tacctgggtg ttggtgttag gatacgtagc tctctgaggt 10800
gaggtctgtg tctcaggagg tgggcctggc acgtttctct cgggcagagc gtgatcccgt 10860
tagggagagg ctctcggtta gggcgctcta tcctgttgct gggaagcgtg ttttctgccg 10920
gtggggctct ttgtgggaat ctccgaccgc ctggggattc ggagctgcag gatttgggtc 10980
aaacagacgc tgggctatcg aagccccgac gccgggccac cgcacgcttc tttgctcagg 11040
tgctggagcc gcctctcggg gcccgaggag ggaccctgga gctgcgcccg cgatctcgct 11100
accgtttaca gctgcgcgcc aggctcaacg gccccaccta ccaaggtccc tggagctcgt 11160
ggtcggaccc aactagggtg gagaccgcca ccgagaccgg tgaggcaagc cccggccgca 11220
ccaaagccgc acagcgcctg cgccagggac tgggcgccgg gtgcgagtgg ggcggggctc 11280
ggagaggggc gaggcgcggg gcgcggagag gggcggggcc ctgaccttgc gggccgacgg 11340
ctgcgcaggt gcccgcagtg cccaggggcg gcgaggggcg gggccagagt aggggctggc 11400
tggatgaggg cggggctccg gcccgggtgg gccgaagtct gacccttttt gtctcctagc 11460
ctggatctcc ttggtgaccg ctctgcatct agtgctgggc ctcagcgccg tcctgggcct 11520
gctgctgctg aggwdgcagt ttcctgcaca ctacaggtac cgcccccgcc aggcaggaga 11580
ctggcggtgg accaggtgga gccgaaggcc tgtaaacagg cattcttggt tcgctctgtg 11640
accccagatc tccgtccacc gcccgtgcgc acctacggct tcgccacttc ctgcacgtca 11700
cctctgggac tcgccgcggc tccttacact ctaacacgcc cactataccg cccacctcga 11760
acagccccgc ctcctgctgc tcacctcggc gactaggcca ccgtccaccc ttcagccaaa 11820
ctgcccactc cacccccatc caatctgccg tcaggtccca cctcctaaac ctagtccaaa 11880
caatggcccc ctttctctag ccctacagac acgacctgac tcactgaaac agaacctctt 11940
gcccctccga tctagcctca cacagccacc agaataatct ttctaaaatg cgcacatgaa 12000
cctatcaatt ccctccttaa atcctttcaa tagctcccag gatatagtcc aggctcccta 12060
tgatgacaag cgaggctctg caaaatcggc ccctgccagc aagcacttca gcctgttcta 12120
ccctgtgcat cagcctttcc aaactggttt tctccacaag tcaggtttta cttctcagtg 12180
cctttgcaca taacgtccgc tctgcctgaa atagcctccc tgtctcacta aacccattca 12240
tctgtaaata actttcttca ccaactctta agaaaccttt cctgaccctc ccccttttgg 12300
ccgctgctct gtgctccaat acagtaccag tagtgtttta ctgtgtttat ttctttatat 12360
atcttcccta ttatacctta cttggtcatc tttaaatcct tagcacagtg cctgccaaaa 12420
agcaggccct taaataaatg ttattaatga gtgactcatt tatgtgggaa gacgaaggct 12480
gaggagaaat gccattggag gctataaaca tgaagggtag aatgcatccc acctccatcc 12540
ttcatctgct gcttctttaa gacttagctc agaagtcatc tcctccagat ggtgtcttct 12600
gatggtcctg ggctggttca ggtgccactc tcatgggcta ccatctccct ctgaacatac 12660
ctctctttga acttttaatc tgcccatacg tatgagcaga ctgaaaggta gcctccatgc 12720
aggtagccag atccaacaaa aaagttggag aagtaggagg aggtggtctg ctgagccagg 12780
catactcagt ttcttttccc caatttacca ggtaagtcac accttctccc ctggagagat 12840
atgagtgagg tcactgaagc ggcctgaaat gttctactga cgtccctcca catggaaata 12900
tgaagctaaa gaatccaggt tctggccggg tgcggtggct cacgcctgta atcccagcac 12960
tttgggaggc cgaggtgggc agatcacaag gtcaggagat caagaccatc ctggctaaca 13020
cagtgaaacc ccatctctac taaaaataca aaaaaaatta gccaggcatg gtggtgggcg 13080
cctgtagtcc cagctactca ggaggctgag gcaggagaat ggtgtgaacc tgagaggcgg 13140
agcttgcagt gagccaagac cgcgccactg cactccagcc tgggccacag agggagactc 13200
catctcaaaa acaaaacaaa acaaaacaaa acaaaacaaa acaaaacaac aaaaaaatgg 13260
aggcccctcc aatacatctt aatccatatt cttcccagat agactaccct ctccagtaac 13320
ttactaataa gctccactga ttttcttcat tcattcattc agctaattta ctgagcacct 13380
actatgggtc agacactatt ctacacccta gaaatacagg agtgaataaa tagacaaact 13440
ctcttttctc ggggaccata tgttctagtg gggtgagaca gacaaaaaga aatatacata 13500
acaatgtaat aaatgtacaa caagtcaaat actataagga aaaatgaagt acagcaggag 13560
atagcctgtc atggggaaag gaagcagcac tacagtggtc ccctcttatc cacagtttca 13620
ctttttgtgg tttcagttac acgcagtcaa ttaaggtctg aaaaaaatta catggaaaat 13680
tccagaaata aacaattcat gtattttaaa ttgtaagtag cacgatgaaa tcacttgcca 13740
tgccgctctg tcccacccgg gatgtaaatc atccctttgt ccagcatctc cacgctgtat 13800
atgctccctg ccccatagtc acttagcagc catcttgctt atcagattga ctcgttgagg 13860
tattgcagag ctgtgttcaa gtaaccctca ttttagttaa taatggcccc aaagtacaag 13920
agtagtgatg ttggcatatt gttctaattg ttctatttta ttagtagtca ttattaatct 13980
cttactgtgc ctaatttata aactttattg taggtatgta cgtatagaaa aaacatctgc 14040
tgggagtctt gaaacatatc ccctgcagat aaagagggac tactgtgttt caggcaaggt 14100
agtcagggat gacatttgaa cagaggccta caatgaggtg agggaatgaa tccagtgttc 14160
cagacagaac aaacattgga gtgtttgagg aaactgcagc accctttgtt gaacctgccc 14220
accactgact tccctcccct acctgaggtt ttcttcttac tctactcccc tgcccaactt 14280
tattgccttg accatgctct tatatctcca agccttactc cccggcctca ctgctccccc 14340

accccaactg ctgtcctcct ccctgccaat ccactgccat ggctcagtct gcttctcttc 14400
cttctccccc aggagactga ggcatgccct gtggccctca cttccagacc tgcaccgggt 14460
cctaggccag taccttaggg acactgcagc cctgagcccg gtgagtgtgc ttccctcccc 14520
tgtgcccacc accaaccctg cctggtactg gatccttgcc ccaacaatac aacttgttca 14580
aggtccttgc cctaccagtg tgccatcccc aggcactacc ccagcactac cccagccctt 14640
ctccttcctg tacagtccag cccctcctcc cacaggatct gctttaatcc agcgcctctc 14700
ctcatctctc ccagcccaag gccacagtct cagatacctg tgaagaagtg gaacccagcc 14760
tccttgaaat cctccccaag tcctcagaga ggactccttt gcccctgtgt tcctcccagg 14820
cccagatgga ctaccgaaga ttgcagcctt cttgcctggg gaccatgccc ctgtctgtgt 14880
gcccacccat ggctgagtca gggtcctgct gtaccaccca cattgccaac cattcctacc 14940
taccactaag ctattggcag cagccttgag gacaggctcc tcactcccag ttccctggac 15000
agagctaaac tctcgagact tctctgtgaa cttccctacc ctacccccac aacacaagca 15060
ccccagacct cacctccatc cccctctgtc tgccctcaca attaggcttc attgcactga 15120
tcttactcta ctgctgctga cataaaacca ggaccctttc tccacaggca ggctcatttc 15180
actaagctcc tcctttactt tctctctcct ctttgatgtc aaacgccttg aaaacaagcc 15240
tccacttccc cacacttccc atttactctt gagactactt caattagttc ccctactaca 15300
ctttgctagt gaaactgccc aggcaaagtg cacctcaaat cttctaattc caagatccaa 15360
taggatctcg ttaatcatca gttcctttga tctcgctgta agatttgtca aggctgacta 15420
ctcacttctc ctttaaattc tttcctacct tggtcctgcc tctttgagta tattagtagg 15480
ttttttttat ttgtttgaga cagggtctca ctctgtcacc caggctgcag tgcaatggcg 15540
cgatctcagc tcactgcaac ctccacctcc gggttcaagc gattcttgtg cctcggcctc 15600
cctagtagct gggattacag gcgcacacca ccacacacag ctaatttttt tttttttttt 15660
tttttttttt ttttagacgg agccttgctc tgttgccaga ctggagtgca gtggcacgat 15720
ctcggctcac tgcaacctct gcctcccggg ttcaagccat tctgcctcag cctcccaagt 15780
agctgggagt acaggcgtct gccaccatgc ctaatttttt tctattttta ggagagaccg 15840
gttttcacca cgttggccag gatggtctcg atatcctgat ctcgtgatcc gcctgcctct 15900
gcctcccaaa gtgctgggat tacaggtgtg acccactgcg cacagcccca gctaatttcc 15960
atatttttag tagagacagg gttttgccat gttgcccagg ctggtcttga actcctaacc 16020
tcgggtgatc cacccacctt ggcctcccaa agtgttagga ttacaggcat gagccactgc 16080
gcccggctga gtgtactagt agttaagaga ataaactaga tctagaatca gagctggatt 16140
caattcctgt ccttcacatt tactagctgt gcaaccttgg gcacataact taatgtcttt 16200
gagccttagt tttttcatct gtaaaacagg gataataaca gcaccccata gagttgtgac 16260
gaggattgag ataatctaag taaagcacag tccctaggac atagtaaatg attcatatat 16320
ccgaactact gttataatta ttccttctta ctctcctctt ctagcatttc ttccaattat 16380
tacagtcctt caagattcca tttcttaaca gtctccaatc ccatctattc tctgccttta 16440
ctatatgttg accattccaa agttcttatc tctagctcag acatctacta cagcactgtg 16500
atgctttatg caactaactg tttacatatc tgtcccctgc tactagattg tgagctcctt 16560
gagggaaagg aacatgattt atttgtcctt ttcccccagc acctagagta gtgcttggtg 16620
catgatagta ggccttcaat aaattttttc taaatgaatg a 16661

<210> 2
<211> 16
<212> DNA
<213> Artificial

<220>
<223> probe

<400> 2
ctgaggwdgc agtttc 16

<210> 3
<211> 17
<212> DNA
<213> Artificial

<220>
<223> probe

<400> 3
gctgaggwdg cagtttc 17

<210> 4
<211> 17
<212> DNA
<213> Artificial

<220>
<223> probe

<400> 4
ctgaggwdgc agtttcc 17


<210> 5
<211> 16
<212> DNA
<213> Artificial

<220>
<223> probe

<400> 5
ctgaggttgc agtttc 16


<210> 6
<211> 17
<212> DNA
<213> Artificial

<220>
<223> probe

<400> 6
gctgaggaag cagtttc 17


<210> 7
<211> 17
<212> DNA
<213> Artificial

<220>
<223> probe

<400> 7
ctgaggtggc agtttcc 17


<210> 8
<211> 21
<212> DNA
<213> Artificial

<220>
<223> primer

<400> 8
tgggccgaag tctgaccctt t 21


<210> 9
<211> 24
<212> DNA
<213> Artificial

<220>
<223>  primer

<400>  9
acagagcgaa ccaagaatgc ctgt                                              24


<210>  10
<211>  17
<212>  DNA
<213>  Artificial

<220>
<223>  probe

<400>  10
ctgccacctc agcagca                                                      17


<210>  11
<211>  14
<212>  DNA
<213>  Artificial

<220>
<223>  probe

<400>  11
aggaaactgc aacc                                                         14


<210>  12
<211>  13
<212>  DNA
<213>  Artificial

<220>
<223>  probe

<400>  12
ggaaactgct tcc                                                          13


<210>  13
<211>  16
<212>  DNA
<213>  Artificial

<220>
<223>  probe

<400>  13
ctgaggaagc agtttc                                                       16

## Description

### Technical Field

The present invention relates to a probe for detecting a polymorphism in the MPL gene and use of the probe.

### Background Art

Chronic myeloproliferative disorders (MPDs) are clonal diseases caused by defects in hematopoietic stem cells. The chronic MPDs collectively refer to diseases such as chronic myelocytic leukemia (CML), polycythemia vera (PV), essential thrombocythemia (ET), and primary myelofibrosis (PMF). It is known that, in some patients with essential thrombocythemia and primary myelofibrosis, due to mutation of the MPL (myeloproliferative leukemia) gene encoding a thrombopoietin receptor, tryptophan (W) as the 515th amino acid of the MPL protein is substituted with leucine (L) or lysine (K) (Non-Patent Document 1). Also, it has been revealed that the substitution with leucine (L) (W515L) is caused by, in the base sequence of the MPL gene shown in SEQ ID NO: 1, the mutation of guanine (g) as the 11535th base into thymine (t), and the substitution with lysine (K) (W515K) is caused by, in the same base sequence, the mutation of thymine (t) as the 11534th base into adenine (a) and guanine (g) as the 11535th base into adenine (a) (Non-Patent Document 1). Therefore, by detecting the presence or absence of these mutations, i.e., polymorphisms, in the MPL gene, diagnosis and the like of essential thrombocythemia and primary myelofibrosis become possible, for example.

On the other hand, as a method for detecting a polymorphism in a gene, various methods have been reported. Examples thereof include a PCR (Polymerase Chain Reaction)-RFLP (Restriction Fragment Length Polymorphism) method.

The PCR-RFLP method is carried out by amplifying a detection target region in a target DNA in a sample by PCR, treating the obtained amplification product with a restriction enzyme, and typing the change in restriction fragment length caused by a polymorphism according to Southern hybridization. When a target mutation is present in the gene, the recognition site of the restriction enzyme disappears. Thus, it is possible to detect the presence or absence of the mutation based on the presence or absence of cleavage, i.e., the change in restriction fragment length.

However, in the PCR-RFLP method, for example, after the PCR, it is necessary to conduct a cumbersome procedure of treating the obtained amplification product with a restriction enzyme and conducting an analysis. Furthermore, in order to treat the obtained amplification product with a restriction enzyme, the amplification product has to be temporarily taken out from a reactor. Thus, there is a risk that the amplification product obtained in a first reaction may be dispersed and mixed with a second reaction that is different from the first reaction. Such problems make the automation of polymorphism detection difficult.

In light of these problems, Tm (Melting Temperature) analysis is attracting attention as a method for detecting a polymorphism in recent years. In Tm analysis, first, using a probe complementary to a region containing a detection target polymorphism, a hybrid (double-stranded nucleic acid) of a nucleic acid to be examined (hereinafter simply referred to as a "test nucleic acid") and the probe is formed. Then, the thus-obtained hybrid is heat-treated, and dissociation (melting) of the hybrid into single-stranded nucleic acids accompanying the temperature rise is detected by measuring a signal such as absorbance. By determining the Tm value based on the result of the detection, the polymorphism is determined. The Tm value becomes higher as the complementarity between the single-stranded nucleic acids of the hybrid becomes higher, and becomes lower as the complementarity between the same becomes lower. Thus, in the case where the polymorphism in a detection target site is X or Y, the Tm value of a hybrid composed of a nucleic acid containing the target polymorphism (e.g., Y) and a probe that is 100% complementary thereto is determined beforehand (this Tm value represents the evaluation standard value). Subsequently, the Tm value of a hybrid composed of the test nucleic acid and the probe is measured (this Tm value represents the measured value). Then, when this measured value is the same as the evaluation standard value, it can be determined that the test nucleic acid shows a perfect match with the probe, i.e., the detection target site in the test nucleic acid is the target polymorphism (Y). On the other hand, when the measured value is lower than the evaluation standard value, it can be determined that the test nucleic acid shows a mismatch with the probe, i.e., the detection target site in the test nucleic acid is the other polymorphism (X). According to such a method, a polymorphism can be detected merely by heat-treating a PCR reaction solution containing the probe and then measuring the signal, for example. Thus, it is possible to automate a detection device.

However, in detection methods utilizing such Tm analysis, it is necessary to determine the difference in a single base from the Tm value, for example. Therefore, in particular, even in the case where a wild-type polymorphism and a mutant-type polymorphism are present together, for example, it is required to detect the presence or absence of mutation accurately.

### Citation List

### Non-Patent Document(s)

[Non-Patent Document 1] Paradanani et al., Blood, 2006, Vol. 108, No. 10, pp. 3472-3476

### Brief Summary of the Invention

### Problem to be Solved by the Invention

With the foregoing in mind, it is an objective of the present invention to provide a probe for detecting a polymorphism, capable of identifying a polymorphism in the MPL gene easily and with high reliability, and use of the probe.

### Means for Solving Problem

In order to achieve the above objective, the present invention provides a probe for detecting a polymorphism in the MPL gene, which is any one of a probe containing an oligonucleotide (P1), a probe containing an oligonucleotide (P1'), a probe containing an oligonucleotide (P2), and a probe containing an oligonucleotide (P2'), wherein:
(P1) is a 9- to 50-mer oligonucleotide composed of a base sequence including the 11535th to 11543rd bases in SEQ ID NO: 1 and having the 11543rd base in its 3' end region;
(P1') is an oligonucleotide composed of a base sequence complementary to the oligonucleotide (P1);
(P2) is a 10- to 50-mer oligonucleotide composed of a base sequence including the 11535th to 11544th bases in SEQ ID NO: 1 and having the 11544th base in its 3' end region; and
(P2') is an oligonucleotide composed of a base sequence complementary to the oligonucleotide (P2).

The present invention also provides a reagent for detecting a polymorphism in the MPL gene, containing the probe of the present invention.

The present invention also provides a method for detecting a polymorphism in the MPL gene, including the step of: detecting a polymorphism in the MPL gene using the probe of the present invention.

According to the probe of the present invention, a polymorphism in the MPL gene can be identified easily and with high reliability, for example. Brief Description of Drawings

FIG. 1 is a graph showing the result of Tm analysis with respect to a reaction solution in which the proportion of wt is 100% in Example 1 of the present invention.
FIG. 2 is a graph showing the result of Tm analysis with respect to a reaction solution in which the proportion of W515L is 50% in Example 1 of the present invention.
FIG. 3 is a graph showing the result of Tm analysis with respect to a reaction solution in which the proportion of W515K is 50% in Example 1 of the present invention.

### Mode for Carrying out the Invention

In the present invention, a detection target polymorphism in the MPL gene is, for example, a polymorphism in at least one of the 11534th base and the 11535th base in the base sequence of the MPL gene shown in SEQ ID NO: 1. The wild-type MPL gene is such that, in the base sequence of the MPL gene shown in SEQ ID NO: 1, the 11534th base (w) is thymine (t) and the 11535th base (d) is guanine (g), and in the MPL protein, the 515th amino acid is tryptophan (515W). Examples of mutant MPL genes include the following two mutants. One is such that, in the base sequence of the MPL gene shown in SEQ ID NO: 1, the 11534th base (w) is thymine (t) and the 11535th base (d) is thymine (t), and in the MPL protein, the 515th amino acid is leucine (L) (W515L). The other is such that, in the base sequence of the MPL gene shown in SEQ ID NO: 1, the 11534th base (w) is adenine (a) and the 11535th base (d) is adenine (a), and in the MPL protein, the 515th amino acid is lysine (W515K). In the MPL gene, when the 11534th or 11535th base is of the above-described mutant types, it can be determined that the subject may have a disease such as essential thrombocythemia or primary myelofibrosis, for example. Hereinafter, the polymorphism in the MPL gene corresponding to 515W also is referred to as the "wild-type polymorphism or normal polymorphism"; the polymorphism in the MPL gene corresponding to W515L also is referred to as a "W515L polymorphism or W515L mutant polymorphism"; and the polymorphism in the MPL gene corresponding to W515K also is referred to as a "W515K polymorphism or W515K mutant polymorphism".

The base sequence of the MPL gene is registered under GenBank Accession No. NG_007525, for example. The base sequence of SEQ ID NO: 1 is the full-length base sequence of the MPL gene, and it corresponds to a region from the 5001st base to the 21661st base in the base sequence of Accession No. NG_007525.

In the present invention, hereinafter, the MPL gene in which the above-described bases are of mutant type are referred to as the "mutant-type MPL genes". Among these mutant MPL genes, the one having the W515L polymorphism is referred to as a "W515L mutant MPL gene", and the one having the W515K polymorphism is referred to as a "W515K mutant MPL gene". Also, the MPL gene having the wild-type polymorphism is referred to as a "wild-type MPL gene" or "normal MPL gene".

In the present invention, the site at which the above-described polymorphisms occur, i.e., the 11534th and 11535th bases in the base sequence of SEQ ID NO: 1 (sense strand), or bases to be paired with the 11534th and 11535th bases in the sense strand in a complementary sequence thereto (antisense strand), is referred to as the "detection target site". In the base sequence of SEQ ID NO: 1 (sense strand) or in the complementary sequence thereto (antisense strand), a region containing the detection target site and to which the probe can hybridize is referred to as a "detection target sequence, detection target region, or hybridization region". Regarding the detection target sequence, the one showing a perfect match with the probe is referred to as a "perfect match sequence", and the one showing a mismatch with the probe is referred to as a "mismatch sequence". In the present invention, a perfect match means that the bases in the detection target site are complementary to bases to be paired therewith in the probe, and preferably means that the detection target sequence is perfectly complementary with the probe. In the present invention, a mismatch means that the bases in the detection target site are not complementary to bases to be paired therewith in the probe, and preferably means that the detection target sequence is perfectly complementary to the probe except for the detection target site.

In the present invention, in the case where the MPL gene is amplified and further, the probe of the present invention is caused to hybridize with the resultant amplification product, a region to be amplified in the MPL gene hereinafter is referred to as an "amplification target region". The amplification target region may be, for example, a region in the sense strand of the MPL gene, a region corresponding thereto in the antisense strand, or both of them. In the present invention, the terms "sense strand" and "antisense strand" encompass, for example, amplification products of the sense strand and amplification products of the antisense strand, respectively.

In the present invention, the ends of a base sequence means the endmost bases on the 5' side and the 3' side in the base sequence. A 5' end region is a region including several bases from the 5' end in a base sequence, and a 3' end region is a region including several bases from the 3' end in a base sequence. The several bases mean, for example, one to ten bases including the base at the end. In the present invention, the Zth base (Z is a positive integer) from an end of a base sequence is a numerical order counted with the base at the end as the first base. For example, the first base from the end means the base at the end, and the second base from the end means the base next to the base at the end.

### <Probe for Detecting Polymorphism>

The probe of the present invention is, as described above, a probe for detecting a polymorphism in the MPL gene, which is any one of a probe containing an oligonucleotide (P1), a probe containing an oligonucleotide (P1'), a probe containing an oligonucleotide (P2), and a probe containing an oligonucleotide (P2'), wherein:
(P1) is a 9- to 50-mer oligonucleotide composed of a base sequence including the 11535th to 11543rd bases in SEQ ID NO: 1 and having the 11543rd base in its 3' end region;
(P1') is an oligonucleotide composed of a base sequence complementary to the oligonucleotide (P1);
(P2) is a 10- to 50-mer oligonucleotide composed of a base sequence including the 11535th to 11544th bases in SEQ ID NO: 1 and having the 11544th base in its 3' end region; and
(P2') is an oligonucleotide composed of a base sequence complementary to the oligonucleotide (P2).

As described above, according to the probe of the present invention, a polymorphism in the MPL gene can be identified easily and with high reliability by Tm analysis, for example. Specifically, for example, even in the case where the MPL gene having a wild-type target polymorphism and the MPL gene having a mutant target polymorphism are present together in a sample or in the case where a plurality of MPL genes with different mutant target polymorphisms are present together in a sample, the kind of polymorphism or the presence or absence of the mutation can be detected easily and with high reliability by the Tm analysis using the probe of the present invention. Therefore, the present invention is particularly useful when applied to a sample containing both a wild-type MPL gene and a mutant MPL gene and a sample containing different mutant MPL genes. Thus, according to the present invention, a polymorphism in the MPL gene can be identified easily and with high reliability, so that, for example, the detection result can be reflected in diagnosis and the like of the above-described diseases. Hence, it can be said that the present invention is very useful in the medical field and the like.

The probe of the present invention is a probe for detecting, in the base sequence of the MPL gene shown in SEQ ID NO: 1, polymorphisms (g/a, g/t) at the 11535th base (d), and/or a polymorphism (t/a) at the 11534th base (w) and polymorphisms (g/a, g/t) at the 11535th base (d). In the base sequence of SEQ ID NO: 1, w is thymine (t) or adenine (a), and d is guanine (g), adenine (a), or thymine (t) (hereinafter the same).

The base length of each of the oligonucleotides (P1) and (P1') is 9- to 50-mer as described above. The base length preferably is 13- to 30-mer, more preferably 15- to 20-mer. The base length of each of the oligonucleotides (P2) and (P2') is 10- to 50-mer as described above. The base length preferably is 13- to 30-mer, more preferably 15- to 20-mer.

Each of the oligonucleotides (P1) and (P2) is, for example, homologous to the sense strand of the MPL gene, and the polymorphism can be checked through hybridization of this oligonucleotide with the antisense strand of the MPL gene. The oligonucleotides (P1) and (P2) each contain the 11535th base (d) in the base sequence of SEQ ID NO: 1, further may contain the 11534th base (w) in the same, and preferably contain both of them.

In each of the oligonucleotides (P1) and (P2), examples of the sequence of "wd" include "tg", "tt", and "aa". When "wd is tg", the oligonucleotide shows a perfect match with a detection target sequence in the antisense strand of the wild-type MPL gene. When "wd is tt", the oligonucleotide shows a perfect match with a detection target sequence in the antisense strand of the W515L mutant MPL gene. When "wd is aa", the oligonucleotide shows a perfect match with a detection target sequence in the antisense strand of the W515K mutant MPL gene. Therefore, the polymorphism in the MPL gene can be detected based on whether or not the oligonucleotide shows a perfect match with the detection target sequence in the MPL gene. Specifically, for example, the oligonucleotide in which "wd is tg" can detect the wild-type polymorphism; the oligonucleotide in which "wd is tt" can detect the W515L mutant polymorphism; and the oligonucleotide in which "wd is aa" can detect the W515K mutant polymorphism. Hereinafter, the oligonucleotide in which "wd is tg" also is referred to as a "wild-type detection probe"; the oligonucleotide in which "wd is tt" also is referred to as a "W515L mutant detection probe"; and the oligonucleotide in which "wd is aa" also is referred to as a "W515K mutant detection probe". By using two kinds or all three kinds of the probes respectively containing these oligonucleotides, it is also possible to detect the wild-type polymorphism, the W515L mutant polymorphism, and the W515K mutant polymorphism in the same reaction system, for example, as will be described below.

The oligonucleotide (P1) has the 11543rd base in its 3' end region. The base preferably is any one of the 1st to 4th bases from the 3' end, more preferably any one of the 1st to 3rd bases from the 3' end, and particularly preferably the 1st base (i.e., the 3' end) or the second base from the 3' end. Examples of the oligonucleotide (P1) include oligonucleotides of SEQ ID NO: 2 and SEQ ID NO: 3. The oligonucleotide (P2) has the 11544th base in its 3' end region. The base preferably is any one of the 1st to 4th bases from the 3' end, more preferably any one of the 1st to 3rd bases from the 3' end, and particularly preferably the 1st base (i.e., the 3' end) or the second base from the 3' end. Examples of the oligonucleotide (P2) include an oligonucleotide of SEQ ID NO: 4. In the base sequences of SEQ ID NOs: 2, 3, and 4, the underlined "wd" corresponds to wd as the detection target site in the MPL gene shown in SEQ ID NO: 1.
ctgaggwdgcagtttc (SEQ ID NO: 2)
gctgaggwdgcagtttc (SEQ ID NO: 3)
ctgaggwdgcagtttcc (SEQ ID NO: 4)

In each of the oligonucleotides (P1) and (P2), the wd is any one of tt, aa, and tg, for example, as described above.

Examples of the oligonucleotide of SEQ ID NO: 2 include an oligonucleotide of SEQ ID NO: 5. In the base sequence of SEQ ID NO: 5, the underlined "tt" corresponds to the detection target site in the sense strand of the W515L mutant MPL gene, and a probe containing the oligonucleotide of SEQ ID NO: 5 can be used as a W515L mutant detection probe. Examples of the oligonucleotide of SEQ ID NO: 3 include an oligonucleotide of SEQ ID NO: 6. In the base sequence of SEQ ID NO: 6, the underlined "aa" corresponds to the detection target site in the sense strand of the W515K mutant MPL gene, and a probe containing the oligonucleotide of SEQ ID NO: 6 can be used as a W515K mutant detection probe. Examples of the oligonucleotide of SEQ ID NO: 4 include an oligonucleotide of SEQ ID NO: 7. In the base sequence of SEQ ID NO: 7, the underlined "tg" corresponds to the detection target site in the sense strand of the wild-type MPL gene, and a probe containing the oligonucleotide of SEQ ID NO: 7 can be used as a wild-type detection probe.
ctgaggttgcagtttc (SEQ ID NO: 5)
gctgaggaagcagtttc (SEQ ID NO: 6)
ctgaggtggcagtttcc (SEQ ID NO: 7)

Each of the oligonucleotides (P1') and (P2') is complementary to the sense strand of the MPL gene, for example, and the polymorphism in the MPL gene can be checked through hybridization of this oligonucleotide with the sense strand of the MPL gene. In each of the oligonucleotides (P1') and (P2'), the complementary base to be paired with the base (w) in the detection target site (the 11534th base in SEQ ID NO: 1) in the sense strand is represented as "w", which is thymine (t) or adenine (a), and the base complementary to the base (d) in the detection target site (the 11535th base in SEQ ID NO: 1) in the sense strand is represented as "h", which is cytosine (c), thymine (t), or adenine (a) (hereinafter the same). The base lengths of the oligonucleotides (P1') and (P2') are the same as those of the oligonucleotides (P1) and (P2), respectively.

The probe of the present invention may be a probe containing any of the above-described oligonucleotides or a probe composed of any of the above-described oligonucleotides, for example.

The probe of the present invention preferably is a labeled probe having a labeling substance. For example, it is preferable that the oligonucleotide is labeled (modified) with the labeling substance. In the oligonucleotide, a site to be labeled with the labeling substance is not particularly limited, and preferably it is the 5' end region or the 3' end region, more preferably the 5' end or the 3' end, for example. As will be described below, in the oligonucleotide, a base to be labeled with the labeling substance preferably is cytosine (c) or guanine (g), for example. The base may be labeled directly with the labeling substance, or alternatively, it may be labeled indirectly with the labeling substance, for example. In the latter case, for example, by labeling any site in a nucleotide residue containing the base, the base can be labeled indirectly with the labeling substance. In the oligonucleotides of SEQ ID NOs: 2 to 7, it is preferable that cytosine (c) at the 3' end is labeled with the labeling substance, for example.

Each of the oligonucleotides (P1) and (P2) preferably has the labeling substance in its 3' end region. Specifically, the labeling substance preferably is located at a position of the 1st to 4th bases from its 3' end. It is more preferable that any base from the 1st to 4th bases from the 3' end, still more preferably any base from the 1st to 3rd bases from the 3' end, and particularly preferably the 2nd base from the 3' end or the base at the 3' end has the labeling substance, for example. In the oligonucleotide (P1), it is preferable that, for example, any of the 11540th to 11543rd bases has the labeling substance, and it is more preferable that the 11543rd base (c) has the labeling substance. In the oligonucleotide (P2), it is preferable that, for example, any of the 11541st to 11544th bases has the labeling substance, and it is more preferable that the 11544th base (c) has the labeling substance.

Each of the oligonucleotides (P1') and (P2') preferably has the labeling substance in its 5' end region. Specifically, the labeling substance preferably is located at a position of the 1st to 4th bases from its 5' end. It is more preferable that any base from the 1st to 4th bases from the 5' end, still more preferably any base from the 1st to 3rd bases from the 5' end, and particularly preferably the 2nd base from the 5' end or the base at the 5' end has the labeling substance, for example. In the oligonucleotide (P1'), it is preferable that, for example, the base complementary to any one of the 11540th to 11543rd bases has the labeling substance, and it is more preferable that the base (c) complementary to the 11543rd base has the labeling substance. In the oligonucleotide (P2), it is preferable that, for example, the base complementary to any one of the 11541st to 11544th bases has the labeling substance, and it is more preferable that the base (c) complementary to the 11544th base has the labeling substance.

The labeling substance is not particularly limited, and preferably is the one that gives off a signal depending on whether the labeled probe is present independently or when it forms a hybrid, for example. The kind of signal is not particularly limited, and examples of the signal include fluorescence and colouring. The colouring may be colour development or colour change, for example. When the signal is fluorescence, examples of a signal value include a fluorescence intensity. When the signal is colouring, examples of a signal value include reflectance, absorbance, and transmittance. The signal may be given off from the labeling substance directly or indirectly, for example.

The labeling substance is not particularly limited, and examples thereof include fluorescent substances such as a fluorophore. Examples of the fluorescent substance include fluorescein, phosphor, rhodamine, and polymethine dye derivatives. Examples of commercially available fluorescent substances include Pacific Blue® (Molecular Probes), BODIPY FL® (Molecular Probes), FluorePrime™ (Amersham Pharmacia), Fluoredite™ (Millipore Corporation), FAM® (ABI), Cy3™ and Cy5™ (Amersham Pharmacia), and TAMRA® (Molecular Probes). The detection conditions for the fluorescent substance are not particularly limited, and can be determined as appropriate depending on the type of fluorescent substance to be used, for example. Specifically, Pacific Blue can be detected at a detection wavelength from 450 to 480 nm, for example; TAMRA can be detected at a detection wavelength from 585 to 700 nm, for example; and BODIPY FL can be detected at a detection wavelength from 515 to 555 nm, for example. When such a labeled probe is used, for example, by detecting fluorescence as the signal and measuring fluorescence intensity as the signal value, hybridization and dissociation can be checked easily based on the change in fluorescence intensity. As described above, as the probes of the present invention, it is possible to use two probes selected from the wild-type detection probe, the W515L mutant detection probe, and the W515K mutant detection probe or all of these probes in the same reaction system. This allows the wild-type polymorphism, the W515L mutant polymorphism, and the W515K mutant polymorphism in the MPL gene to be identified in the same reaction system. In this case, it is preferable that the respective probes have different labeling substances that are detected under different conditions.

Preferably, the labeled probe is, for example, a labeled probe that shows a signal independently and shows no signal when it forms a hybrid, or a labeled probe that shows no signal independently and shows a signal when it forms a hybrid. When the labeling substance is a fluorescent substance, the labeled probe preferably is a probe that is labeled with the fluorescent substance, shows fluorescence independently, and shows reduced (e.g., quenched) fluorescence when it forms a hybrid, for example. Such a phenomenon generally is called fluorescence quenching. Probes utilizing this phenomenon generally are called fluorescence quenching probes. Among these fluorescence quenching probes, a preferred probe is one in which the 3' end or the 5' end of the oligonucleotide is labeled with the fluorescent substance, and the base at the end to be labeled preferably is cytosine (c) or guanine (g). In the case where the base at the end is cytosine (c), the base sequence of the fluorescence quenching probe preferably is designed so that, for example, when the fluorescence quenching probe forms a hybrid with a test nucleic acid, the base to be paired with the labeled cytosine (c) at the end or a base away therefrom by one to three bases in the test nucleic acid is guanine (g). A base away from the base to be paired with cytosine (c) by one base is, for example, a base located next to the base to be paired with cytosine (c). Such a probe generally is called a guanine quenching probe, and is known as a so-called QProbe®. When the guanine quenching probe hybridizes to the test nucleic acid, there occurs a phenomenon that, for example, as the fluorescent substance-labeled cytosine (c) at the end approaches guanine (g) in the test nucleic acid, fluorescence of the fluorescent substance becomes weak, in other words, the fluorescence intensity is reduced. By using the probe such as described above, hybridization and dissociation can be checked easily based on the change in fluorescence intensity, for example. Similarly, in the case where the above-described base at the end is guanine (g), the base sequence of the fluorescence quenching probe preferably is designed so that, for example, when the fluorescence quenching probe forms a hybrid with a test nucleic acid, the base to be paired with the labeled guanine (g) at the end or a base away therefrom by one to three bases in the test nucleic acid is cytosine (c).

In the probe of the present invention, for example, a phosphate group may be added to the 3' end. As will be described below, a test nucleic acid can be prepared by a nucleic acid amplification method such as PCR, for example. At this time, the probe of the present invention may be added to the reaction system of the nucleic acid amplification reaction. In such a case, when the 3' end of the probe has a phosphate group, it is possible to sufficiently prevent the probe itself from being elongated by the nucleic acid amplification reaction. A similar effect can be obtained also by adding a labeling substance such as described above to the 3' end of the probe, for example.

In the detection of the polymorphism using the probe of the present invention, the detection method is by no means limited as long as it is a method utilizing the hybridization of the detection target sequence and the probe. As the method for detecting the polymorphism, the polymorphism detection method of the present invention will be described below.

### <Method for Detecting Polymorphism>

The method for detecting a polymorphism according to the present invention is, as described above, a method for detecting a polymorphism in the MPL gene, including the step of: detecting a polymorphism in the MPL gene using the probe of the present invention.

In the method of the present invention, the detection step preferably includes the steps of:
(A) while changing the temperature of a reaction system containing a test nucleic acid and the probe of the present invention, measuring a signal value indicating the melting state of a hybrid of the test nucleic acid and the probe; and
(B) determining the polymorphism in the test nucleic acid based on the change in the signal value accompanying the temperature change.

The method of the present invention is characterized in that it uses the probe of the present invention, and other configurations, conditions, and the like are not limited to those described below. As described above, the probe of the present invention preferably is a labeled probe. In the present invention, the reaction system is a reaction solution, for example.

As described above, in the method of the present invention, one or two kinds of probes of the present invention may be used, and it is preferable that three kinds of probes of the present invention are used in combination. Specifically, one probe or two or more probes selected from the wild-type detection probe, the W515L mutant detection probe, and the W515K mutant detection probe may be used, and it is preferable to use three of them in combination. By using these probes in combination, it is possible to detect whether the polymorphism at the 11534th and 11535th bases in the MPL gene of SEQ ID NO: 1 is of the wild type, the W515L mutant type, or the W515K mutant type in a single reaction system, for example. The combination of the probes is not particularly limited, and examples thereof include the combination of the W515L mutant detection probe containing the oligonucleotide of SEQ ID NO: 5, the W515K mutant detection probe containing the oligonucleotide of SEQ ID NO: 6, and the wild-type detection probe containing the oligonucleotide of SEQ ID NO: 7.

When two or more kinds of the probes are added to a single reaction system, it is preferable that the respective probes are the labeled probes. It is preferable that the labeled probes respectively are labeled with labeling substances that are detected under different detection conditions. With this configuration, two or more kinds of polymorphisms can be detected easily using the same reaction system by merely changing the detection conditions, for example.

In the present invention, the test nucleic acid may be a single-stranded nucleic acid or a double-stranded nucleic acid. When the test nucleic acid is a double-stranded nucleic acid, step (A) preferably includes a step of dissociating the double-stranded test nucleic acid by heating the reaction system, for example, as will be described below. By dissociating the double-stranded nucleic acid into single-stranded nucleic acids, the probe of the present invention can hybridize with the single-stranded nucleic acid.

In the present invention, the test nucleic acid may be a nucleic acid contained inherently in a sample or an amplification product of the nucleic acid, for example. The latter is preferable because, for example, it allows the detection accuracy to be improved. The amplification product can be prepared by amplifying the nucleic acid in the sample as a template nucleic acid according to a nucleic acid amplification method, for example. The amplification product may be an amplification product obtained by using DNA in the sample as a template or an amplification product obtained by using cDNA synthesized from RNA in the sample as a template, for example. Examples of the RNA in the sample include RNAs such as total RNA and mRNA, and the cDNA can be synthesized from, for example, the RNA such as described above by RT-PCR (Reverse Transcription PCR), for example.

In the case where the test nucleic acid is the amplification product, the method of the present invention may further include the following step (X):
(X) producing the amplification product from a template nucleic acid.
Step (X) preferably is performed prior to step (A). Step (X) may be a step of producing the amplification product from the template nucleic acid in a reaction system in the presence of the probe, for example.

In step (A), it is only necessary that the probe is contained in the reaction sysytem, and the timing of the addition of the probe is not particularly limited. In the case where the test nucleic acid is the amplification product, the reaction system in step (A) may be newly prepared using the amplification product obtained in step (X) and the probe, or may be the reaction system of the amplification reaction in step (X), for example. In the latter case, the probe may be added to the reaction system of the amplification reaction before or during step (X). Alternatively, the probe may be added to the reaction system after step (X).

The method for amplifying the nucleic acid is not particularly limited, and examples thereof include a PCR (Polymerase Chain Reaction) method, a NASBA (Nucleic Acid Sequence Based Amplification) method, a TMA (Transcription-Mediated Amplification) method, and a SDA (Strand Displacement Amplification) method. Among them, the PCR method is preferable. The conditions of the method for amplifying the nucleic acid are not particularly limited, and the method can be carried out using conventionally known techniques.

In the production of the nucleic acid amplification product from the template nucleic acid, it is preferable to use a primer for amplifying a region containing the detection target polymorphism in the MPL gene, for example. The sequence of the primer is not particularly limited as long as a detection target sequence containing the detection target site can be amplified, for example, and the sequence of the primer can be set as appropriate by a conventionally known method, depending on the detection target sequence, sequences in the vicinity thereof, and the like. The length of the primer is not particularly limited, and can be set to a general length. For example, the length of the primer may be 10- to 30-mer.

As the primer, for example, either one of a forward primer (hereinafter also referred to as "F primer") for amplifying the sense strand of the gene and a reverse primer (hereinafter also referred to as "R primer") for amplifying the antisense strand of the gene may be used. It is preferable to use a primer set including a pair composed of these primers. Examples of the F primer and the R primer are shown below. It is to be noted that they are merely illustrative and by no means limit the present invention.

F primer
   5'-tgggccgaagtctgacccttt-3' (SEQ ID NO: 8)
R primer
   5'-acagagcgaaccaagaatgcctgt-3' (SEQ ID NO: 9)

In the reaction system, the amount of the primer to be added is not particularly limited. For example, the amount of one kind of primer to be added in the reaction system is, for example, 0.1 to 2 µmol/l, preferably 0.25 to 1.5 µmol/l, and particularly preferably 0.5 to 1 µmol/l. When a F primer and a R primer are used, the ratio (the molar ratio F : R) between the F primer (F) and R primer (R) to be added is not particularly limited, and preferably is 1 : 0.25 to 1 : 4, more preferably 1 : 0.5 to 1 : 2, for example.

In step (A), the ratio (molar ratio) of the probe to be added relative to the test nucleic acid is not particularly limited. It preferably is 1 or less, more preferably 0.1 or less, because this allows a detection signal to be produced sufficiently. At this time, the amount of the test nucleic acid may be, for example, the total amount of a perfect match nucleic acid having a perfect match sequence and a mismatch nucleic acid having a mismatch sequence or may be the total amount of an amplification product containing a perfect match sequence and an amplification product containing a mismatch sequence. Although the amount of the perfect match nucleic acid in the test nucleic acid generally is unknown, it is preferable that the ratio (molar ratio) of the probe to be added relative to the perfect match nucleic acid (the amplification product containing the perfect match sequence) eventually becomes 10 or less, more preferably 5 or less, and still more preferably 3 or less. Furthermore, the lower limit of the ratio is not particularly limited, and is, for example, 0.001 or more, preferably 0.01 or more, and more preferably 0.1 or more. The ratio of the probe to be added relative to the test nucleic acid may be the molar ratio thereof relative to a double-stranded nucleic acid or relative to a single-stranded nucleic acid, for example.

The amount of the probe to be added in the reaction system is not particularly limited. For example, it is preferable to add one kind of probe so that its concentration is in the range from 10 to 1000 nmol/l, more preferably from 20 to 500 nmol/l. Furthermore, in the reaction system, the molar ratio of the probe relative to the test nucleic acid preferably is, for example, 1 or less, more preferably 0.1 or less, because this allows sufficient signal values to be produced, for example. The ratio of the probe to be added relative to the test nucleic acid may be the molar ratio thereof relative to a double-stranded nucleic acid or relative to a single-stranded nucleic acid, for example.

A sample to which the method of the present invention is applied is not particularly limited, and examples thereof include biological samples. Specific examples of the biological sample include: whole blood; blood cells such as leukocyte cells; oral cells such as oral mucosa; somatic cells such as nail and hair cells; germ cells; sputum; amniotic fluid; paraffin-embedded tissues; urine; gastric juice; and liquid obtained by gastrolavage. In the present invention, a method for collecting the sample, a method for preparing a test nucleic acid from the sample, and the like are not limited, and conventionally known methods can be employed.

The method of the present invention can be utilized in so-called Tm analysis such as described above. The following is an explanation of a Tm value in Tm analysis. For example, when a solution containing a double-stranded DNA is heated, absorbance at 260 nm increases. This is caused by the fact that the hydrogen bonds between the strands composing the double-stranded DNA are broken by the heating, whereby the double-stranded DNA is dissociated into single-stranded DNAs (melting of DNA). Then, when every double-stranded DNA is dissociated into single-stranded DNAs, the absorbance of the solution becomes about 1.5 times as large as the absorbance at the time when the heating was initiated (i.e., the absorbance of the solution containing only the double-stranded DNA), whereby it can be determined that the melting is complete. Based on this phenomenon, the melting temperature (Tm) generally is defined as the temperature at the time when the amount of increase in absorbance reaches 50% of the total amount of increase in absorbance.

In step (A), the measurement of a signal indicating the melting state of a hybrid of the test nucleic acid and the probe may be, for example, the measurement of absorbance at 260 nm as described above or the measurement of a signal of the labeling substance. Specifically, it is preferable that a labeled probe labeled with the labeling substance is used as the probe as described above and that a signal of the labeling substance is measured. The labeled probe may be, for example, a labeled probe that shows a signal independently and shows no signal when it forms a hybrid, or a labeled probe that shows no signal independently and shows a signal when it forms a hybrid. The former probe does not show a signal when it forms a hybrid (double-stranded DNA) with the amplification product and shows a signal when the probe is dissociated from the amplification product by heating. On the other hand, the latter probe shows a signal when it forms a hybrid (double-stranded DNA) with the amplification product, and the signal is reduced (quenched) when the probe is dissociated from the amplification product by heating. Therefore, by detecting a signal of the labeling substance, detection of the progress of the melting of the hybrid, determination of the Tm value, and the like can be achieved, as in the case where absorbance at 260 nm is measured. The signal of the labeling substance may be detected under conditions specific to the signal of the labeling substance, for example. Examples of these detection conditions include an excitation wavelength and a detection wavelength. The labeled probe and the labeling substance are as described above.

Next, the method of the present invention will be described with reference to an illustrative example. The present example is directed to the case where the probe of the present invention is a labeled probe labeled with a fluorescent substance, a template nucleic acid is amplified in the presence of the probe, and the resultant amplification product is used as the test nucleic acid. The method of the present invention is characterized in that the probe of the present invention is used in the method, and other steps and conditions are by no means limited.

First, genomic DNA is isolated from the biological sample. Isolation of the genomic DNA from the biological sample is not particularly limited, and can be achieved by a conventionally known method. As a specific example, the isolation can be achieved using a commercially available genomic DNA isolation kit (GFX Genomic Blood DNA Purification kit™; GE Healthcare Bio-Sciences) or the like, for example.

Next, a reaction solution is prepared by adding the labeled probe to a sample containing the isolated genomic DNA. As the labeled probe, for example, QProbe® is preferable, as described above.

The labeled probe may be added to the sample containing the isolated genomic DNA or may be mixed with the genomic DNA in a solvent, for example. The solvent is not particularly limited, and examples thereof include conventionally known solvents including: buffer solutions such as Tris-HCl; solvents respectively containing KCl, MgCl₂, MgSO₄, glycerol, and the like; and reaction solutions for nucleic acid amplification, such as reaction solutions for PCR.

The timing of the addition of the labeled probe is not particularly limited. For example, the labeled probe may be added before, during, or after the nucleic acid amplification reaction. In particular, it is preferable to add the labeled probe to the reaction solution before the nucleic acid amplification reaction because, for example, it is not necessary to expose the reaction solution to the external environment in order to add the labeled probe and it is possible to carry out the nucleic acid amplification reaction and the measurement of signal values successively. In this case, it is preferable that the 3' end of the labeled probe is modified with the labeling substance or a phosphate group, as described above.

Subsequently, using the isolated genomic DNA as a template, a detection target sequence including a detection target polymorphism is amplified in the presence of the labeled probe by a nucleic acid amplification method such as PCR. Although the following description is directed to the case where PCR is used as the nucleic acid amplification method, the present invention is not limited thereto. Also, conditions for the PCR are not particularly limited, and the PCR can be carried out according to a conventionally known method.

Specifically, the reaction solution containing the genomic DNA, the labeled probe, and the primer is subjected to PCR. The composition of the reaction solution is not particularly limited, and those skilled in the art can set the composition as appropriate. In addition to the genomic DNA, the labeled probe, and the primer, the reaction solution further may contain: a polymerase such as DNA polymerase; nucleoside triphosphate; a buffer solution; any of various kinds of catalysts; and the like, for example. The amount of the labeled probe and the primer to be added in the reaction solution is not particularly limited, and may be in the above-described ranges, respectively, for example.

The DNA polymerase is not particularly limited, and conventionally known polymerases derived from heat-resistant bacteria can be used, for example. As specific examples of such polymerases, *Thermus* aquaticus-derived DNA polymerases (U.S. Patent Nos. 4,889,818 and 5,079,352) (Taq polymerase™), *Thermus thermophilus-derived* DNA polymerase (WO 91/09950) (rTth DNA polymerase), *Pyrococcus furiosus*-derived DNA polymerase (WO 92/9689) (Pfu DNA polymerase: Stratagenes), *Thermococcus litoralis*-derived polymerase (EP-A 455 430 (Vent™): New England Biolabs), and the like, for example, are commercially available. Among them, *Thermus* aquaticus-derived heat-resistant DNA polymerases are preferable.

The amount of the DNA polymerase to be added in the reaction solution is not particularly limited, and is, for example, 1 to 100 U/ml, preferably 5 to 50 U/ml, and more preferably 20 to 40 U/ml. With regard to the unit of activity (U) of DNA polymerases, 1 U generally is defined as the activity for incorporating 10 nmol of total nucleotides into an acid-insoluble precipitate at 74°C in 30 minutes in a reaction solution for activity measurement using activated salmon sperm DNA as a template primer. The composition of the reaction solution for activity measurement is as follows, for example: 25 mmol/l TAPS buffer (pH 9.3, 25°C), 50 mmol/l KCl, 2 mmol/l MgCl₂, 1 mmol/l mercaptoethanol, 200 µmol/l dATP, 200 µmol/l dGTP, 200 µmol/l dTTP, 100 µmol/l [α―³²P] dCTP, and 0.25 mg/mL activated salmon sperm DNA.

The nucleoside triphosphate generally is dNTP (dATP, dCTP, dGTP, and dTTP or dUTP). The amount of dNTP to be added in the reaction solution is not particularly limited, and is, for example, 0.01 to 1 mmol/l, preferably 0.05 to 0.5 mmol/l, and more preferably 0.1 to 0.3 mmol/l.

Examples of the buffer solution include Tris-HCl, Tricine, MES, MOPS, HEPES, and CAPS, and it is possible to use commercially available buffer solutions for PCR and buffer solutions included in commercially available PCR kits.

The reaction solution further may contain heparin, betaine, KCl, MgCl₂, MgSO₄, glycerol, or the like, and the amount of these components to be added may be set within ranges where they do not interfere with the PCR reaction.

The total volume of the reaction solution is not particularly limited, and can be determined as appropriate depending on the device to be used, such as a thermal cycler, and the like, for example. The total volume generally is 1 to 500 µl, preferably 10 to 100 µl.

Next, PCR is conducted. The cycle conditions of the PCR are not particularly limited. As a specific example, for (1) dissociation of a double-stranded DNA as the test nucleic acid into single-stranded DNAs; (2) annealing of the primer to the single-stranded DNA; and (3) elongation of the primer through a polymerase reaction, conditions shown in Table 1 below can be employed, for example. The number of cycles in the PCR is not particularly limited. It preferably is 30 cycles or more with the three steps described in the following items (1) to (3) as one cycle, for example. The upper limit of the total number of cycles is not particularly limited, and is, for example, 100 cycles or less, preferably 70 cycles or less, and more preferably 50 cycles or less. The temperature change in each of the steps can be controlled automatically using a thermal cycler or the like, for example.

**[Table 1]**

| | Temperature (°C) and Time (second) |
|---|---|
| (1) Dissociation into single-stranded DNAs | e.g., 90°C to 99°C, 1 to 120 seconds |
| | preferably, 92°C to 95°C, 1 to 60 seconds |
| (2) Annealing of primer | e.g., 40°C to 70°C, 1 to 300 seconds |
| | preferably, 50°C to 70°C, 5 to 60 seconds |
| (3) Elongation of primer | e.g., 50°C to 80°C, 1 to 300 seconds |
| | preferably, 50°C to 75°C, 5 to 60 seconds |

The amount of the labeled probe to be added in the reaction solution is not particularly limited. For example, it is preferable to add the labeled probe so that its concentration is in the range from 10 to 1000 nmol/l, more preferably from 20 to 500 nmol/l. In the reaction solution, the molar ratio of the labeled probe relative to the test nucleic acid preferably is, for example, 1 or less, more preferably 0.1 or less, because this allows sufficient signal to be produced, for example. The ratio of the labeled probe to be added relative to the test nucleic acid may be the molar ratio thereof relative to a double-stranded nucleic acid or relative to a single-stranded nucleic acid, for example.

Next, dissociation of the obtained amplification product (double-stranded DNA) and hybridization of the labeled probe with a single-stranded DNA obtained through the dissociation are carried out. They can be achieved by, for example, changing the temperature of the reaction solution in the presence of the labeled probe. In this case, it is preferable that the reaction solution to which the labeled probe has been added is subjected to an amplification reaction, after which the temperature of the reaction solution is changed, as described above.

The heating temperature in the dissociation step may be, for example, a temperature at which the double-stranded amplification product can be dissociated into single strands. The heating temperature is not particularly limited, and is, for example, 85°C to 95°C. The heating time is not particularly limited, and generally is 1 second to 10 minutes, preferably 1 second to 5 minutes.

The hybridization of the labeled probe with the dissociated single-stranded DNA can be achieved by, for example, lowering the heating temperature in the dissociation step after completion of the dissociation step. The temperature condition is, for example, 40°C to 50°C. The time period for conducting the treatment at this temperature is not particularly limited, and is, for example, 1 to 600 seconds.

Then, signal values indicating the melting states of the hybrid of the amplification product and the labeled probe are measured while changing the temperature of the reaction solution. Specifically, for example, the reaction solution is heated, in other words, the hybrid of the single-stranded DNA and the labeled probe is heated, and change in signal value accompanying the temperature rise is measured. As described above, in the case where a guanine quenching probe, i.e., a probe in which cytosine (c) at the end is labeled, is used, fluorescence is reduced (or quenched) when the probe hybridizes with the single-stranded DNA, and fluorescence is emitted when the probe is dissociated. Therefore, in this case, for example, the hybrid with reduced (quenched) fluorescence may be heated gradually, and the increase in fluorescence intensity accompanying the temperature rise may be measured.

When measuring the change in fluorescence intensity, the temperature range used in the measurement is not particularly limited. The initiation temperature is, for example, room temperature to 85°C, preferably 25°C to 70°C, and the end temperature is, for example, 40°C to 105°C. The temperature rising rate is not particularly limited, and is, for example, 0.1 to 20 °C/sec., preferably 0.3 to 5 °C/sec.

Next, the Tm value is determined by analyzing the change in signal value. Specifically, from the obtained fluorescence intensities, the amount of change in fluorescence intensity per unit time (―d amount of change in fluorescence intensity/dt or d amount of change in fluorescence intensity/dt) at each temperature is calculated, and the temperature at which the amount of change is largest can be determined as the Tm value. When the labeled probe is a fluorescence quenching probe, the Tm value can be determined in the following manner, for example: the amount of increase in fluorescence intensity is measured, and the temperature at which the amount of increase in fluorescence intensity per unit time (―d amount of increase in fluorescence intensity/dt) is smallest or the temperature at which the amount of increase in fluorescence intensity per unit time (d amount of increase in fluorescence intensity/dt) is largest can be determined as the Tm value. On the other hand, when a probe that shows no signal independently and shows a signal when it forms a hybrid is used as the labeled probe instead of the fluorescence quenching probe, the amount of decrease in fluorescence intensity may be measured, contrary to the case stated above.

For example, in the case where a plurality of labeled probes respectively labeled with labeling substances that are detected at different detection wavelengths are used in place of the labeled probe, the change in signal value may be analyzed at each detection wavelength.

The Tm value can be calculated using MELTCALC software (http://www.meltcalc.com/), which is known conventionally, or the like, for example. Also, the Tm value can be determined by a nearest neighbor method.

Then, based on the Tm value, it is determined whether the 11534th or 11535th base in the base sequence of the MPL gene shown in SEQ ID NO: 1 is of the wild type or the mutant type in the detection target sequence. In the Tm analysis, for example, a perfectly complementary hybrid (match) exhibits a higher Tm value, which indicates dissociation, than a hybrid with one or more different bases (mismatch). Therefore, with regard to the labeled probe, by determining the Tm value of a perfectly complementary hybrid and the Tm value of a hybrid with a difference in one or more bases beforehand, it is possible to determine whether the base in the detection target sequence is of the wild type or the mutant type. Furthermore, as described above, by using the wild-type detection probe, the W515L mutant detection probe, and the W515K mutant detection probe in combination, the type of the polymorphism can be determined based on which of the probes shows the Tm value of the perfectly complementary hybrid, for example.

In the present invention, instead of raising the temperature of the reaction system containing the probe, in other words, heating the hybrid, and then measuring the change in the signal accompanying the temperature rise as described above, the change in the signal at the time of hybrid formation may be measured, for example. That is, when forming a hybrid by lowering the temperature of the reaction system containing the probe, the change in signal accompanying the lowering of the temperature may be measured, for example.

As a specific example, the case where a labeled probe that shows a signal independently and shows no signal when it forms a hybrid (e.g., a guanine quenching probe) is used will be described. In this case, the labeled probe emits fluorescence when the single-stranded DNA and the labeled probe are dissociated, and the fluorescence is reduced (or quenched) when the temperature is lowered to allow the labeled probe to form a hybrid. Therefore, for example, the temperature of the reaction solution may be lowered gradually, and the decrease in fluorescence intensity accompanying the temperature lowering may be measured. On the other hand, when a labeled probe that shows no signal independently and shows a signal when it forms a hybrid is used, the labeled probe does not emit fluorescence when the single-stranded DNA and the labeled probe are dissociated, and the labeled probe emits fluorescence when the temperature is lowered to allow the labeled probe to form a hybrid. Therefore, for example, the temperature of the reaction solution may be lowered gradually, and the increase in fluorescence intensity accompanying the temperature lowering may be measured.

### < Reagent for Detection Polymorphism>

The reagent for detecting a polymorphism according to the present invention is a reagent for detecting a polymorphism in the MPL gene, containing the probe of the present invention. The reagent of the present invention is characterized in that it contains the probe of the present invention, and other configurations and conditions are by no means limited.

The reagent may contain one or two kinds of the probes, and preferably contains three kinds of the probes, for example. Specifically, the reagent may contain one or two probes selected from the wild-type detection probe, the W515L mutant detection probe, and the W515K mutant detection probe, and preferably contains three of them. The combination of the probes of the present invention is not particularly limited, and examples thereof include the combination of the W515L mutant detection probe containing the oligonucleotide of SEQ ID NO: 5, the W515K mutant detection probe containing the oligonucleotide of SEQ ID NO: 6, and the wild-type detection probe containing the oligonucleotide of SEQ ID NO: 7.

The reagent of the present invention further may contain a primer or a primer set for amplifying a region including the detection target site in the MPL gene. Examples of the primer include those described above.

The reagent of the present invention further may contain components necessary for the nucleic acid amplification reaction, for example. Specific examples of such components include: polymerases such as DNA polymerases; nucleoside triphosphate; buffer solutions; and various kinds of catalysts, such as those described above. In the polymorphism detection reagent of the present invention, the respective components may be contained in the same container or separate containers, for example.

The reagent of the present invention also can be referred to as a probe kit for use in the detection of the polymorphism in the MPL gene, for example. In the kit of the present invention, the respective components may be contained in the same container or separate containers, for example. The polymorphism detection kit of the present invention further may include instructions for use.

The primer for amplifying the MPL gene according to the present invention is as described above, and is at least one of the primers composed of the oligonucleotides of SEQ ID NOs: 8 to 9. An amplification method according to the present invention is a method for amplifying the MPL gene, including the step of: amplifying the MPL gene with a nucleic acid in a sample as a template in a reaction system using the primer for amplifying the MPL gene according to the present invention. A method for detecting an amplification product according to the present invention is a method for detecting an amplification product of the MPL gene, including the step of: amplifying the MPL gene by the method for amplifying the MPL gene according to the present invention using the primer of the present invention. The detection method according to the present invention preferably further includes the step of: detecting the amplification product of the MPL gene using the probe of the present invention, for example. Regarding the primer of the present invention and the methods using the primer, the above descriptions can be referred to.

Next, examples of the present invention will be described. It is to be noted, however, the present invention is by no means limited by the following examples. In the following examples, "%" means "w/v%" unless otherwise stated.

### Examples

### [Example 1]

In the present example, polymorphisms in the MPL gene were detected by carrying out Tm analysis in the presence of a wild-type plasmid and mutant-type plasmids.

A wild-type plasmid (wt), a mutant-type plasmid (W515L), and a mutant-type plasmid (W515K) were produced. The wt was a double-stranded plasmid obtained through insertion of a partial sequence (from the 11391st to 11711st bases in SEQ ID NO: 1) of the wild-type MPL gene of SEQ ID NO: 1 in which the 11534th base w was thymine (t) and the 11535th base d was guanine (g). The W515L was a double-stranded plasmid obtained through insertion of a partial sequence (from the 11391st to 11711st bases in SEQ ID NO: 1) of the W515L mutant MPL gene in which the 11535th base d was mutated to thymine (t). The W515K was a plasmid obtained through insertion of a partial sequence (from the 11391st to 11711st bases in SEQ ID NO: 1) of the W515K mutant MPL gene in which the 11534th base w was mutated to adenine (a) and the 11535th base d was mutated to adenine (a). Then, the wt was mixed with the W515L or W515K at predetermined mixing ratios. Thus, three kinds of nucleic acid samples shown below were prepared.

(Nucleic Acid Sample)

| Nucleic acid sample | Mixing ratio of respective plasmids | | |
|---|---|---|---|
| | wt | W515L | W515K |
| wt 100% | 100% | 0% | 0% |
| W515L 50% | 50% | 50% | 0% |
| W515K 50% | 50% | 0% | 50% |

1 µl (1 × 10⁴ copies/µl) of each nucleic acid sample and 49 µl of a reaction reagent shown in Table 2 below were added to a tube, thus providing a PCR reaction solution. With respect to the thus-obtained PCR reaction solution, PCR and Tm analysis were carried out using a fully-automated SNP analyzer (i-densy®, ARKRAY, Inc.). PCR was carried out in the following manner: the PCR reaction solution was first treated at 95°C for 60 seconds, then was subjected to 50 cycles of treatment with a treatment at 95°C for 1 second and at 62°C for 15 seconds as one cycle, and further treated at 95°C for 1 second and 40°C for 60 seconds. Subsequently, Tm analysis was carried out by heating the PCR reaction solution from 40°C to 75°C at a temperature rising rate of 1°C/3 seconds and measuring the change in fluorescence intensity with time at detection wavelengths specific to the fluorescent substances used in the respective probes.

**[Table 2]**

| | |
|---|---|
| (Composition of PCR reaction solution: unit µl) distilled water | 32.27 |
| 1 mol/l Tris-HCl (pH 8.6) | 1.25 |
| 20% BSA | 0.50 |
| 10% NaN₃ | 0.23 |
| 80% glycerol | 8.125 |
| 1 mol/l MgCl₂ | 0.075 |
| 1 mol/l KCl | 1.25 |
| 2.5 mmol/l dNTP | 4 |
| 100 µmol/l F primer | 0.25 |
| 100 µmol/l R primer | 0.5 |
| 100 µmol/l W515L mutant detection probe | 0.1 |
| 100 µmol/l W515K mutant detection probe | 0.1 |
| 100 µmol/l wild-type detection probe | 0.1 |
| 5 U/µl Gene Taq (NIPPON GENE) | 0.25 |
| Total | 49 µl |

As the F primer and the R primer, primers having the following sequences were used, respectively.
F primer (SEQ ID NO: 8)
   5'-tgggccgaagtctgacccttt-3'
R primer (SEQ ID NO: 9)
   5'-acagagcgaaccaagaatgcctgt-3'

As the probes, probes having the following sequences were used, respectively.
W515L mutant detection probe 1 (SEQ ID NO: 5)
   5'-ctgaggTTgcagtttc-(TAMRA)-3'
W515K mutant detection probe 1 (SEQ ID NO: 6)
   5'-gctgaggAAgcagtttc-(BODIPY FL)-3'
Wild-type detection probe 1 (SEQ ID NO: 7)
   5'-ctgaggTGgcagtttcc-(Pacific Blue)-3'

The sequences of the W515L mutant detection probe 1, the W515K mutant detection probe 1, and the wild-type detection probe 1 each show a perfect match with the antisense strand of the MPL gene. In each of the sequences, bases indicated with capital letters correspond to the 11534th to 11535th bases (wd) in SEQ ID NO: 1. The 3' end of each of the probes was labeled with a fluorescent substance. TAMRA was detected at a wavelength of 445 to 480 nm, BODIPY FL was detected at a wavelength of 520 to 555 nm, and Pacific Blue was detected at a wavelength of 585 to 700 nm.

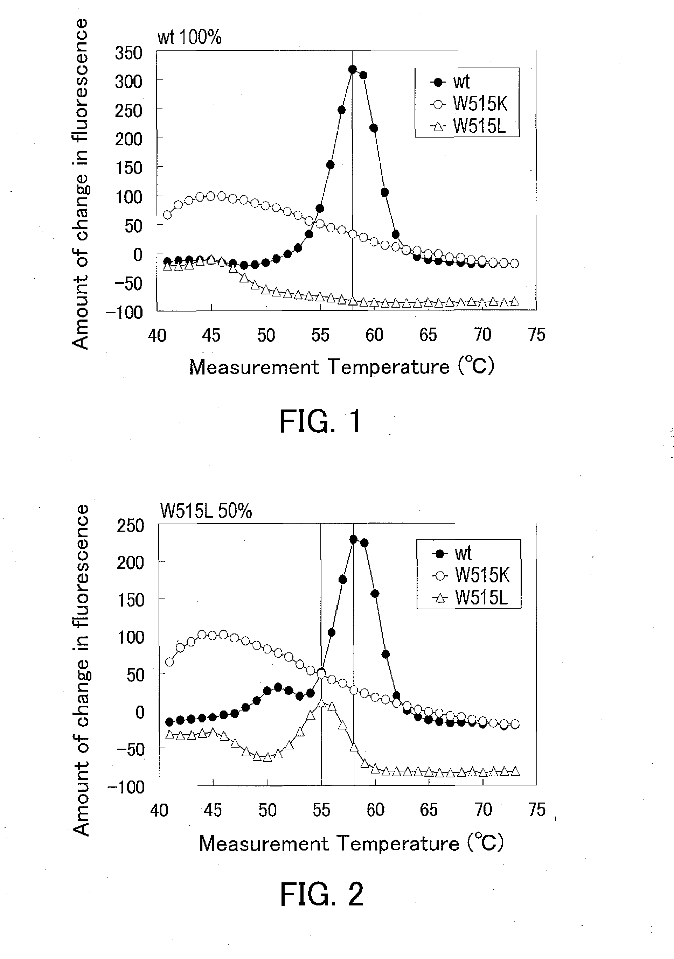

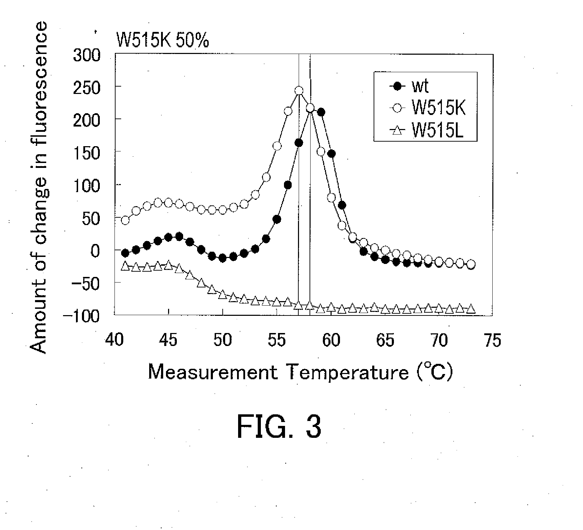

The results thereof are shown in FIGs. 1 to 3. FIGs. 1 to 3 are graphs showing the Tm analysis results and indicate the change in fluorescence intensity accompanying the temperature rise. FIG. 1 shows the result obtained with regard to the nucleic acid sample of wt 100%, FIG. 2 shows the result obtained with regard to the nucleic acid sample of W515L 50%, and FIG. 3 shows the result obtained with regard to the nucleic acid sample of W515K 50%. The horizontal axis indicates the temperature (°C) at the time of measurement. The vertical axis indicates the change in fluorescence intensity (hereinafter also referred to as the "amount of change in fluorescence"), and the unit thereof is "d amount of increase in fluorescence intensity/dt". In FIGs. 1 to 3, a filled circle (●) indicates the detection result obtained by the wild-type detection probe, an open circle (o) indicates the detection result obtained by the W515K mutant detection probe, and an open triangle (Δ) indicates the detection result obtained by the W515L mutant detection probe. In the case where the formed hybrid is a perfect match hybrid, the Tm serving as the evaluation standard value is as follows: the Tm value of the wt is 58°C; the Tm value of the W515L is 55°C; and the Tm value of the W515K is 57°C.

As can be seen from FIG. 1, with regard to the sample in which the proportion of the wt was 100%, a peak was observed only at the Tm value of the wt. Furthermore, as can be seen from FIG. 2, with regard to the sample in which the proportion of the W515L was 50% and the proportion of the wt was 50%, peaks were observed both at the Tm value of the wt and at the Tm value of the W515L. As can be seen from FIG. 3, with regard to the sample in which the proportion of the W515K was 50% and the proportion of the wt was 50%, peaks were observed both at the Tm value of the wt and at the Tm value of the W515K. These results demonstrate that, by using the wild-type detection probe, the W515L mutant detection probe, and the W515K mutant detection probe according to the present example, it is possible to detect the polymorphisms of the wild-type and the two kinds of mutants regardless of which of the polymorphisms is present.

### [Comparative Example 1]

In the present example, polymorphisms in the MPL gene were detected in the same manner as in Example 1, except that the probes shown below were used as the probes. In the following sequences, P indicates phosphorylation at the 3' end.

Wild-type detection probe 2 (SEQ ID NO: 10)
   5'-(TAMRA)-ctgcCAcctcagcagca-P-3'
W515L mutant detection probe 2 (SEQ ID NO: 11)
   5'-aggaaactgcAacc-(TAMRA)-3'
W515K mutant detection probe 2 (SEQ ID NO: 12)
   5'-ggaaactgcTTcc-(TAMRA)-3'
W515K mutant detection probe 3 (SEQ ID NO: 13)
   5'-(TAMRA)-ctgaggAAgcagtttc-P-3'

When these probes were used, unlike the results obtained in Example 1, even in the case where the plasmids to be detected were present, the fluorescence was not quenched so that no peak was observed in the amount of change in fluorescence.

### Industrial Applicability

As specifically described above, according to the present invention, a polymorphism in the MPL gene can be identified easily and with high reliability by Tm analysis, for example. The present invention is particularly useful when applied to a sample containing both the wild-type MPL gene and the mutant MPL gene or a sample containing different mutant MPL genes. Thus, according to the present invention, a polymorphism in the MPL gene can be identified easily and with high reliability, so that, for example, the detection result can be reflected in diagnosis and treatment of the above-described diseases. Hence, it can be said that the present invention is very useful in the medical field and the like.

While the present invention has been described with reference to illustrative embodiments and examples, it is to be understood that changes and modifications that may become apparent to those skilled in the art may be made without departing from the scope of the present invention.

This application claims priority from Japanese Patent Application No. 2009-251429 filed on October 30, 2009. The entire disclosure of the Japanese Patent Application is incorporated herein by reference.

## Claims

1. A probe for detecting a polymorphism in the MPL gene, the probe being any one of a probe comprising an oligonucleotide (P1), a probe comprising an oligonucleotide (P1'), a probe comprising an oligonucleotide (P2), and a probe comprising an oligonucleotide (P2'), wherein:
(P1) is a 9- to 50-mer oligonucleotide composed of a base sequence including the 11535th to 11543rd bases in SEQ ID NO: 1 and having the 11543rd base in its 3' end region;
(P1') is an oligonucleotide composed of a base sequence complementary to the oligonucleotide (P1);
(P2) is a 10- to 50-mer oligonucleotide composed of a base sequence including the 11535th to 11544th bases in SEQ ID NO: 1 and having the 11544th base in its 3' end region; and
(P2') is an oligonucleotide composed of a base sequence complementary to the oligonucleotide (P2).

2. The probe according to claim 1, wherein, in the oligonucleotides (P1) and (P2), the base sequence composed of the 11534th to 11535th bases in the base sequence of SEQ ID NO: 1 is any one of tt, aa, and tg.

3. The probe according to claim 1, wherein,
in the oligonucleotide (P1), the 11543rd base is any one of the 1st to 4th bases from the 3' end, and
in the oligonucleotide (P2), the 11544th base is any one of the 1st to 4th bases from the 3' end.

4. The probe according to claim 1, wherein
the oligonucleotide (P1) is any one of the oligonucleotides of SEQ ID NOs: 2 and 3, and
the oligonucleotide (P2) is an oligonucleotide of SEQ ID NO: 4:
ctgaggwdgcagtttc (SEQ ID NO: 2)
gctgaggwdgcagtttc (SEQ ID NO: 3)
ctgaggwdgcagtttcc (SEQ ID NO: 4).

5. The probe according to claim 4, wherein
the oligonucleotide of SEQ ID NO: 2 is the oligonucleotide of SEQ ID NO: 5,
the oligonucleotide of SEQ ID NO: 3 is the oligonucleotide of SEQ ID NO: 6, and
the oligonucleotide of SEQ ID NO: 4 is the oligonucleotide of SEQ ID NO: 7:
ctgaggttgcagtttc (SEQ ID NO: 5)
gctgaggaagcagtttc (SEQ ID NO: 6)
ctgaggtggcagtttcc (SEQ ID NO: 7).

6. The probe according to claim 1, wherein the probe is a labeled probe having a fluorescent labeling substance.

7. The probe according to claim 6, wherein
each of the oligonucleotides (P1) and (P2) has the fluorescent labeling substance in its 3' end region, and
each of the oligonucleotides (P1') and (P2') has the fluorescent labeling substance in its 5' end region.

8. The probe according to claim 6, wherein
each of the oligonucleotides (P1) and (P2) has the fluorescent labeling substance at a position of the 1st to 4th bases from its 3' end, and
each of the oligonucleotides (P1') and (P2') has the fluorescent labeling substance at a position of the 1st to 4th bases from its 5' end.

9. The probe according to claim 6, wherein,
in the oligonucleotide (P1), the 11543rd base has the fluorescent labeling substance,
in the oligonucleotide (P1'), the base complementary to the 11543rd base in SEQ ID NO: 1 has the fluorescent labeling substance,
in the oligonucleotide (P2), the 11544th base has the fluorescent labeling substance, and
in the oligonucleotide (P2'), the base complementary to the 11544th base in SEQ ID NO: 1 has the fluorescent labeling substance.

10. The probe according to claim 1, wherein the probe is a probe for use in Tm analysis.

11. A reagent for detecting a polymorphism in the MPL gene, the reagent comprising:
the probe according to claim 1.

12. The reagent according to claim 11, further comprising:
a primer for amplifying a region including the polymorphism to be detected in the MPL gene.

13. A method for detecting a polymorphism in the MPL gene, the method comprising the step of:
detecting a polymorphism in the MPL gene using the probe according to claim 1.

14. The method according to claim 13, wherein the detection step comprises the steps of:
(A) while changing the temperature of a reaction system containing a test nucleic acid and the probe according to claim 1, measuring a signal value indicating the melting state of a hybrid of the test nucleic acid and the probe; and
(B) determining the polymorphism in the test nucleic acid based on the change in the signal value accompanying the temperature change.

15. The method according to claim 14, wherein the reaction system comprises two or more kinds of probe.

16. The method according to claim 15, wherein, as the probes, the reaction system comprises two probes selected from the group consisting of a probe comprising an oligonucleotide of SEQ ID NO: 2, a probe comprising an oligonucleotide of SEQ ID NO: 3, and a probe comprising an oligonucleotide of SEQ ID NO: 4, and
the base sequence wd in SEQ ID NO: 2, the base sequence wd in SEQ ID NO: 3, and the base sequence wd in SEQ ID NO: 4 are different from one another and are tt, aa, or tg:
ctgaggwdgcagtttc (SEQ ID NO: 2)
gctgaggwdgcagtttc (SEQ ID NO: 3)
ctgaggwdgcagtttcc (SEQ ID NO: 4).

17. The method according to claim 15, wherein, as the probes, the reaction system comprises a probe comprising an oligonucleotide of SEQ ID NO: 2, a probe comprising an oligonucleotide of SEQ ID NO: 3, and a probe comprising an oligonucleotide of SEQ ID NO: 4, and
the base sequence wd in SEQ ID NO: 2, the base sequence wd in SEQ ID NO: 3, and the base sequence wd in SEQ ID NO: 4 are different from one another and are tt, aa, or tg:
ctgaggwdgcagtttc (SEQ ID NO: 2)
gctgaggwdgcagtttc (SEQ ID NO: 3)
ctgaggwdgcagtttcc (SEQ ID NO: 4).

18. The method according to claim 16, wherein
the oligonucleotide of SEQ ID NO: 2 is the oligonucleotide of SEQ ID NO: 5,
the oligonucleotide of SEQ ID NO: 3 is the oligonucleotide of SEQ ID NO: 6, and
the oligonucleotide of SEQ ID NO: 4 is the oligonucleotide of SEQ ID NO: 7:
ctgaggttgcagtttc (SEQ ID NO: 5)
gctgaggaagcagtttc (SEQ ID NO: 6)
ctgaggtggcagtttcc (SEQ ID NO: 7).

19. The method according to claim 15, wherein the two or more kinds of probes are labeled probes having different labeling substances, respectively.

20. The method according to claim 14, wherein, in step (A), the test nucleic acid is an amplification product obtained by amplifying a template nucleic acid.

21. The method according to claim 20, further comprising the step of:
(X) producing an amplification product from the template nucleic acid,
wherein step (X) is a step of producing the amplification product from the template nucleic acid in the reaction system in the presence of the probe, and
in step (A), the signal value is measured while changing the temperature of the reaction system in step (X).

## Amended claims

### Amended claims under Art. 19.1 PCT

**1.** (Amended) A probe for detecting a polymorphism in the MPL gene, the probe being any one of a probe comprising an oligonucleotide (P1), a probe comprising an oligonucleotide (P1'), a probe comprising an oligonucleotide (P2), and a probe comprising an oligonucleotide (P2'), wherein:
(P1) is a 9- to 50-mer oligonucleotide composed of a base sequence including the 11535th to 11543rd bases in SEQ ID NO: 1, having the 11543rd base in its 3' end region, and having a fluorescent labeling substance at a position of the 1st to 4th bases from its 3' end;
(P1') is an oligonucleotide composed of a base sequence complementary to the oligonucleotide (P1) and having a fluorescent labeling substance at a position of the 1st to 4th bases from its 5' end;
(P2) is a 10- to 50-mer oligonucleotide composed of a base sequence including the 11535th to 11544th bases in SEQ ID NO: 1, having the 11544th base in its 3' end region, and having a fluorescent labeling substance at a position of the 1st to 4th bases from its 3' end; and
(P2') is an oligonucleotide composed of a base sequence complementary to the oligonucleotide (P2) and having a fluorescent labeling substance at a position of the 1st to 4th bases from its 5' end.

**2.** The probe according to claim 1, wherein, in the oligonucleotides (P1) and (P2), the base sequence composed of the 11534th to 11535th bases in the base sequence of SEQ ID NO: 1 is any one of tt, aa, and tg.

**3.** The probe according to claim 1, wherein,
in the oligonucleotide (P1), the 11543rd base is any one of the 1st to 4th bases from the 3' end, and
in the oligonucleotide (P2), the 11544th base is any one of the 1st to 4th bases from the 3' end.

**4.** The probe according to claim 1, wherein
the oligonucleotide (P1) is any one of the oligonucleotides of SEQ ID NOs: 2 and 3, and
the oligonucleotide (P2) is an oligonucleotide of SEQ ID NO: 4:
ctgaggwdgcagtttc (SEQ ID NO: 2)
gctgaggwdgcagtttc (SEQ ID NO: 3)
ctgaggwdgcagtttcc (SEQ ID NO: 4).

**5.** The probe according to claim 4, wherein
the oligonucleotide of SEQ ID NO: 2 is the oligonucleotide of SEQ ID NO: 5,
the oligonucleotide of SEQ ID NO: 3 is the oligonucleotide of SEQ ID NO: 6, and
the oligonucleotide of SEQ ID NO: 4 is the oligonucleotide of SEQ ID NO: 7:
ctgaggttgcagtttc (SEQ ID NO: 5)
gctgaggaagcagtttc (SEQ ID NO: 6)
ctgaggtggcagtttcc (SEQ ID NO: 7).

**6.** (Canceled)

**7.** (Canceled)

**8.** (Canceled)

**9.** (Amended) The probe according to claim 1, wherein,
in the oligonucleotide (P1), the 11543rd base has the fluorescent labeling substance,
in the oligonucleotide (P1'), the base complementary to the 11543rd base in SEQ ID NO: 1 has the fluorescent labeling substance,
in the oligonucleotide (P2), the 11544th base has the fluorescent labeling substance, and
in the oligonucleotide (P2'), the base complementary to the 11544th base in SEQ ID NO: 1 has the fluorescent labeling substance.

**10.** The probe according to claim 1, wherein the probe is a probe for use in Tm analysis.

**11.** A reagent for detecting a polymorphism in the MPL gene, the reagent comprising:
the probe according to claim 1.

**12.** The reagent according to claim 11, further comprising:
a primer for amplifying a region including the polymorphism to be detected in the MPL gene.

**13.** A method for detecting a polymorphism in the MPL gene, the method comprising the step of:
detecting a polymorphism in the MPL gene using the probe according to claim 1.

**14.** The method according to claim 13, wherein the detection step comprises the steps of:
(A) while changing the temperature of a reaction system containing a test nucleic acid and the probe according to claim 1, measuring a signal value indicating the melting state of a hybrid of the test nucleic acid and the probe; and
(B) determining the polymorphism in the test nucleic acid based on the change in the signal value accompanying the temperature change.

**15.** The method according to claim 14, wherein the reaction system comprises two or more kinds of probe.

**16.** The method according to claim 15, wherein, as the probes, the reaction system comprises two probes selected from the group consisting of a probe comprising an oligonucleotide of SEQ ID NO: 2, a probe comprising an oligonucleotide of SEQ ID NO: 3, and a probe comprising an oligonucleotide of SEQ ID NO: 4, and
the base sequence wd in SEQ ID NO: 2, the base sequence wd in SEQ ID NO: 3, and the base sequence wd in SEQ ID NO: 4 are different from one another and are tt, aa, or tg:
ctgaggwdgcagtttc (SEQ ID NO: 2)
gctgaggwdgcagtttc (SEQ ID NO: 3)
ctgaggwdgcagtttcc (SEQ ID NO: 4).

**17.** The method according to claim 15, wherein, as the probes, the reaction system comprises a probe comprising an oligonucleotide of SEQ ID NO: 2, a probe comprising an oligonucleotide of SEQ ID NO: 3, and a probe comprising an oligonucleotide of SEQ ID NO: 4, and
the base sequence wd in SEQ ID NO: 2, the base sequence wd in SEQ ID NO: 3, and the base sequence wd in SEQ ID NO: 4 are different from one another and are tt, aa, or tg:
ctgaggwdgcagtttc (SEQ ID NO: 2)
gctgaggwdgcagtttc (SEQ ID NO: 3)
ctgaggwdgcagtttcc (SEQ ID NO: 4).

**18.** The method according to claim 16, wherein
the oligonucleotide of SEQ ID NO: 2 is the oligonucleotide of SEQ ID NO: 5,
the oligonucleotide of SEQ ID NO: 3 is the oligonucleotide of SEQ ID NO: 6, and
the oligonucleotide of SEQ ID NO: 4 is the oligonucleotide of SEQ ID NO: 7:
ctgaggttgcagtttc (SEQ ID NO: 5)
gctgaggaagcagtttc (SEQ ID NO: 6)
ctgaggtggcagtttcc (SEQ ID NO: 7).

**19.** The method according to claim 15, wherein the two or more kinds of probes are labeled probes having different labeling substances, respectively.

**20.** The method according to claim 14, wherein, in step (A), the test nucleic acid is an amplification product obtained by amplifying a template nucleic acid.

**21.** The method according to claim 20, further comprising the step of:
(X) producing an amplification product from the template nucleic acid,
wherein step (X) is a step of producing the amplification product from the template nucleic acid in the reaction system in the presence of the probe, and
in step (A), the signal value is measured while changing the temperature of the reaction system in step (X).
